(19) Europäisches Patentamt
European Patent Office
Office européen des brevets

(11) Numéro de publication: **0 248 735 B1**

(12) **FASCICULE DE BREVET EUROPEEN**

(45) Date de publication du fascicule du brevet: **23.05.90**

(51) Int. Cl.⁵: **C07D 487/04,** A61K 31/505 // (C07D487/04, 239:00, 235:00)

(21) Numéro de dépôt: **87401253.7**

(22) Date de dépôt: **04.06.87**

(54) **Nouvelles 2-thiazolyl imidazo /1,2-a/ pyrimidines et leurs sels, leur procédé de préparation, leur application à titre de médicaments et les compositions les renformant.**

(30) Priorité: **04.06.86 GB 8613591**

(43) Date de publication de la demande: **09.12.87 Bulletin 87/50**

(45) Mention de la délivrance du brevet: **23.05.90 Bulletin 90/21**

(84) Etats contractants désignés: **AT BE CH DE ES FR GR IT LI LU NL SE**

(56) Documents cités:
**EP-A- 0 104 104**
**EP-A- 0 197 230**

(73) Titulaire: **ROUSSEL-UCLAF, 35, boulevard des Invalides, F-75007 Paris(FR)**

(72) Inventeur: **Deacon, Robert Michael John, 26A Ashford Road, Swindon Wiltshire(GB)**
Inventeur: **Gardner, Colin Robert, Foxlea Hermitage Road Cold Ash, Newbury Berkshire(GB)**
Inventeur: **Gillespie, Roger John, 22 Wentworth Park Freshbrook, Swindon SN5 8QX Wiltshire(GB)**
Inventeur: **Tully Wilfred Roger, Abbey Cottage§ 1 Grove Court, The Waterloo§ Cirencester§Glos§GL7 2PZ(GB)**

(74) Mandataire: **Vieillefosse, Jean-Claude et al, Département des Brevets ROUSSEL UCLAF B.P no 9, F-93230 Romainville(FR)**

**EP 0 248 735 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouvelles 2-thiazolyl imidazo [1,2-a] pyrimidines et leurs sels, ainsi que le procédé de préparation, l'application à titre de médicaments de ces nouveaux produits et les compositions les renfermant.

L'invention a pour objet de nouvelles 2-thiazolyl imidazo [1,2-a] pyrimidines répondant à la formule (I) :

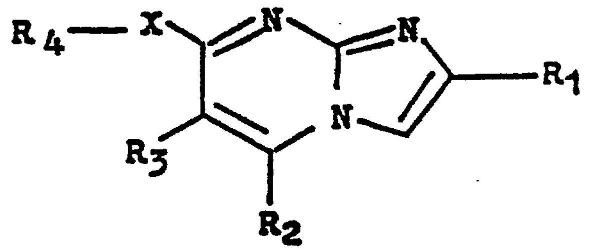

(I)

dans laquelle $R_1$ représente un radical 2-thiazolyl non substitué ou substitué soit par un ou deux radicaux alcoyles renfermant de1 à 3 atomes de carbone, ces radicaux pouvant être eux-mêmes non substitués ou substitués par un ou plusieurs atomes de fluor, soit par un groupe $-CO_2Alk$ dans lequel Alk représente un radical alcoyle renfermant de 1 à 3 atomes de carbone,
$R_2$ et $R_3$, identiques ou différents, représentent un atome d'hydrogène, un radical alcoyle renfermant de 1 à 3 atomes de carbone, un radical alcényle renfermant de 2 à 5 atomes de carbone, ou $R_2$ et $R_3$ forment ensemble un radical alcoylène linéaire renfermant de 3 à 5 atomes de carbone,
X représente un atome d'oxygène ou de soufre et $R_4$ représente un radical alcoyle renfermant de 1 à 3 atomes de carbone, ainsi que leurs sels d'addition avec les acides minéraux ou organiques.

Dans la formule générale (I) et dans ce qui suit :
- le terme radical alcoyle renfermant de 1 à 3 atomes de carbone désigne par exemple un radical méthyle, éthyl ou propyle linéaire ou ramifié. Parmi les radicaux alcoyles renfermant de 1 à 3 atomes de carbone substitués par un ou plusieurs atomes de fluor, on peut citer notamment les radicaux fluorométhyle, difluorométhyl et trifluorométhyl,
-le terme radical alcényle renfermant de 2 à 5 atomes de carbone désigne par exemple un radical vinyle, allyle ou butényle,
- le terme radical alcoylène renfermant de 3 à 5 atomes de carbone désigne par exemple un radical propylène, butylène ou pentaméthylène.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléique, fumarique, succinique, tartrique, citrique, oxalique, glyoxylique, aspartique, alcanesulfoniques tels que l'acide méthanesulfonique et arylsulfoniques, tels que l'acide benzènesulfonique.

Parmi les produits, objet de l'invention, on peut citer notamment les dérivés répondant à la formule (I) ci-dessus, ainsi que leurs sels d'addition avec les acides, caractérisés en ce que dans ladite formule (I), $R_1$ représente un radical thiazol-2-yl, 4-méthylthiazol-2-yl, 4,5-diméthylthiazol-2-yl, 4-éthylthiazol-2-yl, 4-trifluorométhylthiazol-2-yl ou 4-éthoxycarbonylthiazol-2-yl.

Parmi ces derniers, on peut citer les dérivés répondant à la formule (I) ci-dessus, ainsi que leurs sels d'addition avec les acides, caractérisés en ce que dans ladite formule (I), $R_2$ et $R_3$, identiques ou différents représentent un radical alcoyle renfermant de 1 à 3 atomes de carbone ou un radical allyle ou $R_2$ et $R_3$ ensemble représentent le radical $(CH_2)_4$ et X représente un atome d'oxygène.

Parmi les produits, objets de l'invention, on retient tout particulièrement ceux dont les noms suivent :
- la 6-éthyl-7-méthoxy 5-méthyl 2-(4-méthylthiazol-2-yl)imidazo [1,2-a] pyrimidine,
- la 6-éthyl-7-méthoxy 5-méthyl 2-(thiazol-2-yl) imidazo[1,2-a] pyrimidine,
et leurs sels d'addition avec les acides.

L'invention a également pour objet un procédé de préparation des nouvelles 2-thiazolyl imidazo [1,2-a] pyrimidines telles que définies par la formule (I) ci-dessus, ainsi que de leurs sels d'addition avec les acides, caractérisé en ce que l'on traite un produit de formule (II) :

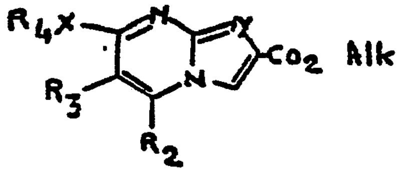

(II)

dans laquelle $R_2$, $R_3$, $R_4$ et X ont la signification déjà indiquée, par un agent basique puis acidifie pour obtenir un produit de formule (III) :

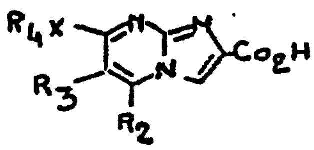

(III)

dans laquelle $R_2$, $R_3$, $R_4$ et X ont la signification déjà indiquée, produit de formule (III) que l'on traite par le N,N'-carbonyldiimidazole pour obtenir un produit de formule (IV) :

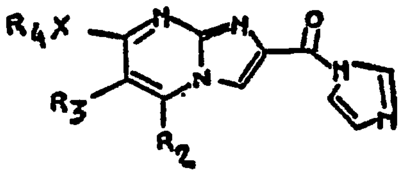

(IV)·

dans laquelle $R_2$, $R_3$, $R_4$ et X ont la signification déjà indiquée produit de formule (IV) que l'on traite par l'ammoniac, pour obtenir un produit de formule (V):

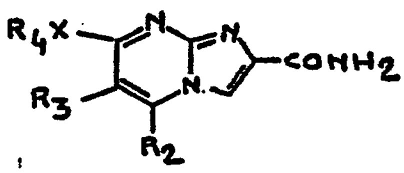

(V)

dans laquelle $R_2$, $R_3$, $R_4$ et X ont la signification déjà indiquée puis fait réagir le produit de formule (V) avec le réactif de Lawesson ou 2,4-bis-(4-méthoxyphényl) 1,3-dithia 2,4-diphosphétane 2,4-disulphide pour obtenir un produit de formule (VI) :

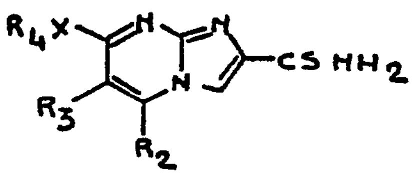

(VI)

dans laquelle $R_2$, $R_3$, R4 et X ont la signification déjà indiquée, puis fait réagir le produit de formule (VI) avec un produit de formule (VII) :

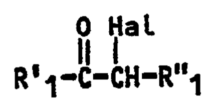

dans laquelle Hal représente un atome d'halogène, $R'_1$ et $R''_1$ identiques ou différents représentent soit un atome d'hydrogène, soit un radical alcoyle renfermant de 1 à 3 atomes de carbone non substitué ou substitué par un ou plusieurs atomes de fluor, soit par un radical $-CO_2Alk$ dans lequel Alk est défini comme ci-dessus, traite l'intermédiaire obtenu par un agent basique pour obtenir un produit de formule (I) que l'on isole et si désiré salifie.

Dans des conditions préférentielles de mise en oeuvre de l'invention, le procédé de préparation ci-dessus décrit est caractérisé en ce que :
- l'agent basique que l'on fait réagir sur le produit de formule (II) est par exemple le carbonate de potassium et la réaction est effectuée au sein d'un mélange aqueux d'un solvant organique tel que le méthanol en chauffant à température de reflux du mélange réactionnel ;
- la réaction du produit de formule (III) avec le N,N'-carbonyldiimidazole est effectuée au sein d'un solvant organique, par exemple dans le diméthyl formamide ;
- le traitement à l'ammoniac du produit de formule (IV) est effectué dans un solvant organique tel que le chloroforme ;
- la réaction du produit de formule (V) avec le réactif de Lawesson est effectué au sein d'un solvant organique tel que le tétrahydrofuranne à température de reflux du milieu réactionnel ;
- dans la formule (VII) Hal représente de préférence un atome de chlore ;
- la réaction due produit de formule (VI) avec le produit de formule (VII) est effectuée au sein d'un solvant organique tel que l'ethanol et l'intermédiaire obtenu est soumis à un agent basique tel que l'ammoniaque concentré.

Lorsque le substituant en position 4 du cycle thiazolique est fortement électronégatif comme par exemple le radical $CF_3$, on soumet l'intermédiaire obtenu au cours de la réaction du produit de formule (VI) et

due produit de formule (VIII) à une forte déshydratation. On opère avantageusement en isolant cet intermédiaire et en le traitant par un agent de déshydratation puissant tel que l'anhydride trifluoroacétique en présence d'un base telle que la triéthylamine au sein d'un solvant aprotique tel que le dichlorométhane.

Lorsque le cycle thiazolique n'est pas substitué en position 4, on utilise au départ un haloaldéhyde dialkylacétal de préférence à l'haloaldéhyde seul.

Dans ce cas on effectue "in situ" l'hydrolyse de l'acétal par addition d'acide aqueux au milieu réactionnel.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques ; ils agissent notamment sur les récepteurs de benzodiazépines du cerveau. Ils peuvent être utilisés comme tranquilisants légers dans le traitement de l'anxiété, de l'obèsité et des troubles cognitifs.

Ces propriétés sont illustrées plus loin dans la partie expérimentale.

Ces propriétés justifient l'utilisation des nouvelles 2-thiazolyl imidazo [1,2-a] pyrimidines de formule (I), ainsi que de leurs sels, pharmaceutiquement acceptables, à titre de médicaments.

La présente invention a ainsi également pour objet l'application à titre de médicaments des nouvelles 2-thiazolyl imidazo [1,2-a] pyrimidines telles que définies par la formule générale (I) ainsi que de leurs sels d'addition avec les acides pharmaceutiquement acceptables.

Parmi les médicaments, objet de l'invention, on retient notamment les médicaments, caractérisés en ce qu'ils sont constitués par les nouvelles 2-thiazolyl imidazo [1,2-a] pyrimidines répondant à la formule (I) dans laquelle $R_1$ représente un radical thiazol-2-yl, 4-méthylthiazol-2-yl, 4,5-diméthylthiazol-2-yl, 4-éthylthiazol-2-yl, 4-trifluorométhylthiazol-2-yl ou 4-éthoxycarbonylthiazol-2-yl ainsi que par leurs sels d'addition avec les acides pharmaceutiquement acceptables.

Parmi les médicaments, objet de l'invention, on retient tout particulièrement ceux répondant à la formule (I), caractérisés en ce que dans ladite formule (I), $R_2$ et $R_3$, identiques ou différents, représentent un radical alcoyle renfermant de 1 à 3 atomes de carbone ou un radical allyle ou $R_2$ et $R_3$ ensemble représentent le radical $(CH_2)_4$ et X représente un atome d'oxygène.

Parmi les médicaments préférés de l'invention, on retient tout particulièrement :
- la 6-éthyl 7-méthoxy 5-méthyl 2-(4-méthylthiazol-2-yl) imidazo [1,2-a] pyrimidine,
- la 6-éthyl 7-méthoxy 5-méthyl 2-(thiazol-2-yl) imidazo [1,2-a] pyrimidine,

ainsi que leurs sels d'addition avec les acides pharmaceutiquement acceptables.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, des dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médicine humaine, comme par exemple les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions, pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) sont des produits connus et peuvent être préparés selon le procédé décrit dans le brevet européen 0 104 104.

Les exemples suivants illustrent l'invention sans toutefois la limiter.

**Exemple 1** : 6-éthyl-7-méthoxy 5-méthyl 2-(4-méthylthiazol-2-yl)imidazo [1,2-a] pyrimidine.

**Stade A** : : Acide 6-éthyul 7-méthoxy 5-méthylimidazo [1,2-a] pyrimidine 2-carboxylique.

On chauffe 6 heures au reflux 120 g de 6-éthyl-7- méthoxy-5-méthylimidazo [1,2-a] pyrimidine-2-carboxylate d'éthyle et 120 g de carbonate de potassium dans 1200 cm3 de méthanol et 600 cm3 d'eau. On évapore le méthanol, ajoute 1500 cm3 d'eau et acidifie le milieu réactionnel jusqu'à pH = 1 à l'aide d'acide chlorhydrique concentré. On filtre le précipité, le lave à l'eau, le sèche à 80⌧C sous pression réduite

et receuille 87,43 g d'acide 6-éthyl-7-méthoxy-5-méthylimidazo [1,2-a] pyrimidine-2-carboxylique.

**Stade B** : 6-éthyl-7-méthoxy 5-méthylimidazo [1,2-a] pyrimidine-2-carboximidazolide.

On ajoute 73,9 g de N,N'-carbonyldiimidazole dans une solution de 87,31 g de l'acide obtenu au stade précédent dans 1000 cm3 de diméthyl formamide et agite 2 heures à température ambiante. On filtre le produit obtenu, le lave au diméthylformamide puis à l'éther, le sèche et obtient 104,71 g de produit attendu.

**Stade C** : 6-éthyl-7-méthoxy 5-méthylimidazo [1,2-a] pyrimidine-2-carboxamide.

On fait barboter pendant 2 heures de l'ammoniac dans une solution de 93 g du produit obtenu au stade précédent dans 1400 cm3 de chloroforme puis maintient 16 heures sous agitation à température ambiante. On lave la solution avec de la lessive de soude, sèche et élimine les solvants sous pression réduite. On reprend le résidu dans l'éther, sèche et récupère 68,7 g de produit attendu. F = 256-259°C.

**Stade D** : 6-éthyl-7-méthoxy 5-méthylimidazo [1,2-a] pyrimidine-2-thiocarboxamide.

On chauffe au reflux pendant 4 heures 20 g du produit obtenu au stade précédent et 25,4 g de réactif de Lawesson dans 470 cm3 de tétrahydrofuranne. On refroidit, filtre, lave le produit obtenu au tétrahydrofuranne puis à l'éther, sèche et obtient 12,06 g de produit attendu. F = 248-259°C.

**Stade E** : 6-éthyl-7-méthoxy-5-méthyl-2-(4-méthylthiazol-2-yl)imidazo [1,2-a] pyrimidine.

On chauffe au reflux 4,3 g du produit obtenu au stade précédent et 3,18 g de chloroacétone dans 300 cm3 d'éthanol. Après 5 heures de chauffage, on ajoute de nouveau 3,18 g de chloroacétone puis poursuit le chauffage pendant 24 heures. On évapore le solvant, dissout le résidu dans 2 litres d'eau, alcalinise avec de l'ammoniaque concentrée et extrait au chloroforme. On élimine le solvant sous pression réduite, purifie par chromatographie sur silice, en éluant au chloroforme et obtient après recristallisation dans l'acétate d'éthyle 3,37 g de produit attendu. F = 184-186°C.

**Exemples 2 à 10** :

En utilisant une méthode analogue à celle décrite à l'exemple 1 mais en partant des composés correspondants de formule (II) dans laquelle $R_2$, $R_3$, $R_4$ et X ont les significations indiquées dans le tableau I ci-après, on a préparé le produits des exemples 2 à 10 (voir tableau I ci-après). La microanalyse, les analyses spectrométriques, les rendements et les points de fusion des composés sont aussi donnés dans le tableau I.**Exemple 2** : 6-éthyl-2-(4éthylthiazol-2-yl)-7-méthoxy-5-méthylimidazo [1,2a] pyrimidine.
**Exemple 3** : 2-(4,5-diméthylthiazol-2-yl-6-éthyl-7-méthoxy-5-méthylimidazo [1,2-a] pyrimidine.
**Exemple 4** : Ethyl 2-(6-éthyl 7-méthoxy-5-méthylimidazo [1,2-a] pyrimidin-2-yl)-thiazol-4-carboxylate.
**Exemple 5** : 6-éthyl-5-méthyl-2-(4-méthylthiazol-2-yl)-7-méthylthio-imidazo [1,2-a] pyrimidine.
**Exemple 6** : 6-éthyl-7-méthoxy-5-méthyl-2-(4-trifluorométhylthiazol-2-yl)-imidazo [1,2-a] pyrimidine.
**Exemple 7** : 5-méthoxy-2-(4-méthylthiazol-2-yl)-6,7,8,9-tétrahydroimidazo [1,2-a] quinazoline.
**Exemple 8** : 6-allyl-7-méthoxy-5-méthyl-2-(4-méthylthiazol-2-yl)imidazo [1,2-a] pyrimidine.
**Exemple 9** : 7-méthoxy-5-méthyl-2-(4-méthylthiazol-2-yl)-6-propylimidazo [1,2-a] pyrimidine.
**Exemple 10** : 6-éthyl-7-méthoxy-5-méthyl-2-(thiazol-2-yl)imidazo [1,2-a] pyrimidine.

TABLEAU I

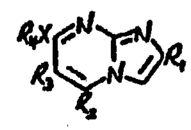

| Exemple | $R_1$ | $R_2$ | $R_3$ | $R_4$ | X | Rendt % | IR(KBr)cm$^{-1}$ | F (°C) | Formule | M.Wt. | Analyse Calc./Tr C | H | N | S |
|---|---|---|---|---|---|---|---|---|---|---|---|---|---|---|
| 1 | 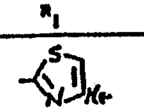 | Me | Et | Me | O | 68 | 3085,2975,2950,2920,1640 | 184-186 | $C_{14}H_{16}N_4OS$ | 288.38 | 58.31<br>58.43 | 5.59<br>5.59 | 19.43<br>19.25 | 11.12<br>11.02 |
| 2 | 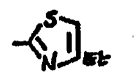 | Me | Et | Me | O | 47 | 3160,2965,1637 | 121-122 | $C_{15}H_{18}N_4OS$ | 302.40 | 59.58<br>59.54 | 6.00<br>6.07 | 18.53<br>18.49 | 10.60<br>10.58 |
| 3 | 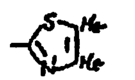 | Me | Et | Me | O | 79 | 2970,2920,1630 | 221-222 | $C_{15}H_{18}N_4OS$ | 302.40 | 59.58<br>59.65 | 6.00<br>6.01 | 18.53<br>18.55 | 10.60<br>10.70 |
| 4 | 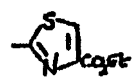 | Me | Et | Me | O | 60 | 2985,2960,2940,1733,1641 | 211-215 | $C_{16}H_{18}N_4O_3S$ | 346.41 | 55.48<br>55.54 | 5.24<br>5.25 | 16.17<br>16.20 | 9.25<br>9.26 |
| 5 | 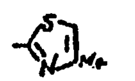 | Me | Et | Me | S | 64 | 3070,2960,2925,2870,1610 | 151-152 | $C_{14}H_{16}N_4S_2$ | 304.45 | 55.23<br>55.06 | 5.30<br>5.26 | 18.40<br>18.42 | 21.06<br>20.92 |
| 6 | 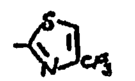 | Me | Et | Me | O | 80 | 3140,2990,2950,1640 | 179-181 | $C_{14}H_{13}N_4OSF_3$ | 342.35 | 49.12<br>49.08 | 3.83<br>3.80 | 16.37<br>16.47 | 9.36<br>9.51 |
| 7 | 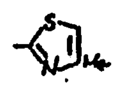 | $-CH_2CH_2CH_2CH_2-$ | | Me | O | 39 | 3060,2935,1640 | 260-263 | $C_{15}H_{16}N_4OS$ | 300.39 | 59.98<br>59.72 | 5.37<br>5.38 | 18.65<br>18.66 | 10.67<br>10.53 |
| 8 | 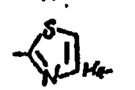 | Me | allyl | Me | O | 76 | 3150,3100,2940,1630 | 149-150 | $C_{15}H_{16}N_4OS$ | 300.39 | 59.98<br>59.91 | 5.37<br>5.38 | 18.65<br>18.73 | 10.67<br>10.60 |
| 9 | 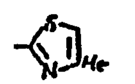 | Me | Pr | Me | O | 71 | 3070,2960,2930,2870,1635 | 160-161 | $C_{15}H_{18}N_4OS$ | 302.40 | 59.58<br>59.56 | 6.00<br>6.00 | 18.53<br>18.67 | 10.60<br>10.57 |
| 10 | 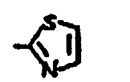 | Me | Et | Me | O | 37 | 2960,2950,2930,1635 | 142-144 | $C_{13}H_{14}N_4OS$ | 274.34 | 56.91<br>56.81 | 5.14<br>5.16 | 20.42<br>20.14 | 11.69<br>11.51 |

**Exemple 11** :

On a préparé des comprimés correspondants à la formulation suivante :
- Composés de l'exemple 1 .................................. 20 mg
- Excipient q.s. pour un comprimé terminé à ................ 150mg
(Détail de l'excipient : lactose, amidon, talc, stéarate de magnésium).

**Exemple 12** :

On a préparé des comprimés correspondants ä la formulation suivante :
- Composés de l'exemple 10 ................................. 20 mg
- Excipient q.s. pour un comprimé terminé à ................ 150mg
(Détail de l'excipient : lactose, amidon, talc, stéarate de magnésium).

**ACTIVITE PHARMACOLOGIQUE**

Les produits de formule (I) agissent sur les récepteurs des benzodiazépines du cerveau et certains d'entre eux peuvent être utilisés comme tranquilisants légers.

L'affinité des composés pour les récepteurs des benzodiazépines a été évaluée en utilisant un radioligand [$^3$H] flunitrazépam et la méthode de Squires et Braestrup (Nature, 1977, 266, 732) modifiée.

Les valeurs indiquées dans le tableau II ci-après sont les concentrations nanomolaires du produit testé qui inhibent dans une proportion de 50% la liaison spécifique de 0,6 nanomoles de [$^3$H] flunitrazépam dans des préparations de membranes de cerveaux antérieurs de rats [($CI_{50}$) en nanamoles].

TABLEAU II

| Exemple | Liaison aux récepteurs $CI_{50}$ Nm |
|---|---|
| 1 | 112 |
| 2 | 220 |
| 3 | 2,500 |
| 4 | 2,800 |
| 5 | 80 |
| 6 | 2,000 |
| 7 | 22 |
| 8 | 100 |
| 9 | 100 |
| 10 | 200 |

Les propriétés agonistes inverses des benzodiazépines sont indiquées par les tests suivants :

a) Seuil de potentialisation des convulsions induites par une stimulation auditive chez des souris $DBA_2$ (Lensen et al - Life Sciences 33, 393-9 (1983)).

Des souris $DBA_2$ sont soumises à une stimulation auditive. Les caractéristiques du seuil de convulsion (myoclonie, course) sont potentialisées par les composés étudiés jusqu'à obtention de convulsions toniques. Une dose efficace 50 ($DE_{50}$) est calculée.
- Composé de l'exemple 1 $DE_{50}$ =3 mg/kg IP
(30 minutes avant le test).

b) Potentialisation des convulsions induites par l'injection sous-cutanée de Leptazol à des souris $CD_1$.

On choisit une dose de Laptazol qui produit 10-20% de convulsions chez des souris $CD_1$ non traitées. Les produits actifs augmentent le pourcentage des convulsions et la $DE_{50}$ est calculée par la méthode de Lichfield et Wilcoxon (J. Pharmacol. Exp. Ther. (1949) 96, 99).
- Composé de l'exemple 1 : $DE_{50}$ = 50 mg/kg IP
(30 minutes avant le test).

c) Induction de contractions due muscle suprahyoide chez des rats Wistar anesthésiés à l'uréthane (James & Gardner - Europ. J. Pharmacol. 113, 233-8 (1985)).

Des rats Wistar prétraités au nialamide sont anesthésiés à l'uréthane et leurs muscles suprahyoïdes sont dégagés. Ces muscles se contractent spontanément et les produits actifs augmentent l'amplitude et/ou le nombre des contractions.
- Composé de l'exemple 1 : DEM = 10 mg/kg IP.

**Revendications pour les Etats contractants BE, CH, DE, FR, IT, LI, LU, NL, SE**

1.- Nouvelles 2-thiazolyl imidazo [1,2-a] pyrimidines répondant à la formule (I) :

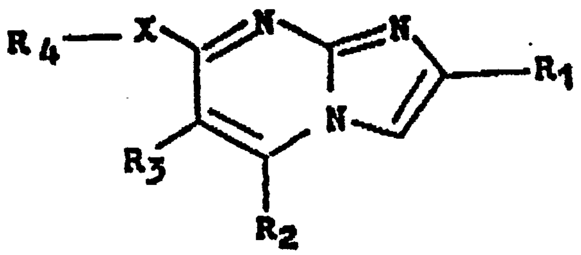

(I)

dans laquelle $R_1$ représente un radical 2-thiazolyl non substitué ou substitué soit par un ou deux radicaux alcoyles renfermant de1 à 3 atomes substitués par un ou plusieurs atomes de fluor, soit par un groupe -$CO_2$Alk dans lequel Alk représente un radical alcoyle renfermant de 1 à 3 atomes de carbone,
$R_2$ et $R_3$, identiques ou différents, représentent un atome d'hydrogène, un radical alcoyle renfermant de 1 à 3 atomes de carbone, un radical alcényle renfermant de 2 à 5 atomes de carbone, ou $R_2$ et $R_3$ forment ensemble un radical alcoylène linéaire renfermant de 3 à 5 atomes de carbone,
X représente un atome d'oxygène ou de soufre et $R_4$ représente un radical alcoyle renfermant de 1 à 3 atomes de carbone, ainsi que leurs sels d'addition avec les acides minéraux ou organiques.

2.- Nouvelles 2-thiazolyl imidazo [1,2-a] pyrimidines telles que définies à la revendication 1 et leurs sels d'addition avec les acides, caractérisés en ce que dans ladite formule (I), $R_1$ représente un radical thiazol-2-yl, 4-méthylthiazol-2-yl, 4,5-diméthylthiazol-2-yl, 4-éthylthiazol-2-yl, 4-trifluorométhylthiazol-2-yl ou 4-éthoxycarbonylthiazol-2-yl.

3.- Nouvelles 2-thiazolyl imidazo [1,2-a] pyrimidines telles que définies à la revendication 1 ou 2 et leurs sels d'addition avec les acides, caractérisées en ce que dans ladite formule (I), $R_2$ et $R_3$, identiques ou différents représentent un radical alcoyle renfermant de 1 à 3 atomes de carbone ou un radical allyle ou $R_2$ et $R_3$ ensemble représentent le radical $(CH_2)_4$ et X représente un atome d'oxygène.

4.- L'un quelconque des nouvelles 2-thiazolyl imidazo [1,2-a] pyrimidines répondant à la formule (I) de la revendication 1 dont les noms suivent :
- la 6-éthyl 7-méthoxy 5-méthyl 2-(4-méthylthiazol-2-yl) imidazo [1,2-a] pyrimidine,
- la 6-éthyl 7-méthoxy 5-méthyl 2-(thiazol-2-yl) imidazo [1,2-a] pyrimidine,
et leurs sels d'addition avec les acides.

5.- Procédé de préparation des nouvelles 2-thiazolyl imidazo [1,2-a] pyrimidines telles que définies par la formule (I) de la revendication 1, ainsi que de leurs sels d'addition avec les acides caractérisé en ce que l'on traite un produit de formule (II) :

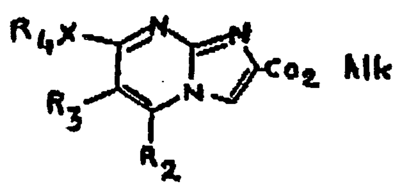

(II)

dans laquelle $R_2$, $R_3$, $R_4$ et X ont la signification déjà indiquée, par un agent basique puis acidifie pour obtenir un produit de formule (III) :

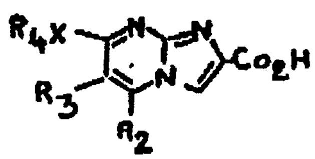

(III)

dans laquelle $R_2$, $R_3$, $R_4$ et X ont la signification déjà indiquée, produit de formule (III) que l'on traite par le N,N'-carbonyldiimidazole pour obtenir un produit de formule (IV) :

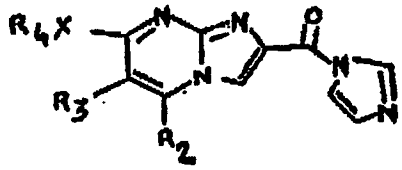

(IV)

dans laquelle $R_2$, $R_3$, $R_4$ et X ont la signification déjà indiquée, produit de formule (IV) que l'on traite par l'ammoniac, pour obtenir un produit de formule (V) :

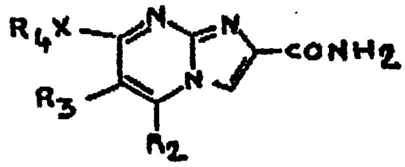

(V)

dans laquelle $R_2$, $R_3$, $R_4$ et X ont la signification déjà indiquée puis fait réagir le produit de formule (V) avec le réactif de Lawesson ou 2,4-bis-(4-méthoxyphényl)-1,3-dithia-2,4-diphosphétane-2,4-disulphide pour obtenir un produit de formule (VI) :

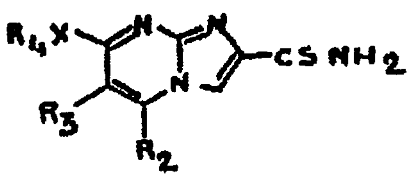

(VI)

dans laquelle $R_2$, $R_3$, $R_4$ et X ont la signification déjà indiquée, puis fait réagir le produit de formule (VI) avec un produit de formule (VII) :

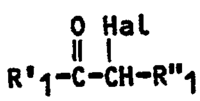

dans laquelle Hal représente un atome d'halogène, $R'_1$ et $R''_1$ identiques ou différents représentent soit un atome d'hydrogène, soit un radical alcoyle renfermant de 1 à 3 atomes de carbone non substitué ou substitué par un ou plusieurs atomes de fluor, soit par un radical $-CO_2Alk$ dans lequel Alk est défini comme ci-dessus, traite l'intermédiaire obtenu par un agent basique pour obtenir un produit de formule (I) que l'on isole et si désiré salifie.

6.- Procédé de préparation selon la revendication 7, caractérisé en ce que :
- l'agent basique que l'on fait réagir sur le produit de formule (II) est par exemple le carbonate de potassium et la réaction est effectuée au sein d'un mélange aqueux d'un solvent organique tel que le méthanol en chauffant à température de reflux du mélange réactionnel ;
- la réaction du produit de formule (III) avec le N,N'-carbonyldiimidazole est effectuée au sein d'un solvant organique, par exemple dans le diméthyl formamide ;
- le traitement à l'ammoniac du produit de formule (IV) est effectué dans un solvant organique tel que le chloroforme ;
- la réaction du produit de formule (V) avec le réactif de Lawesson est effectué au sein d'un solvant organique tel que le tétrahydrofuranne à température de reflux du milieu réactionnel ;
- dans la formule (VII) Hal représente de préférence un atome de chlore ;
- la réaction du produit de formule (VI) avec le produit de formule (VII) est effectuée au sein d'un solvant organique tel que l'éthanol et l'intermédiaire obtenu est soumis à un agent basique tel que l'ammoniaque concentrée.

7.- Médicaments, caractérisés en ce qu'ils sont constitués par les nouvelles 2-thiazolyl imidazo [1,2-a] pyrimidines telles que définies par la formule (I) de la revendication 1, ainsi que par leurs sels d'addition avec les acides pharmaceutiquement acceptables.

8.- Médicaments, caractérisés en ce qu'ils sont constitués par les nouvelles 2-thiazolyl imidazo [1,2-a] pyrimidines telles que définies à l'une quelconque des revendications 2 ou 3, ainsi que par leurs sels d'addition avec les acides pharmaceutiquement acceptables.

9.- Médicaments, caractérisés en ce qu'ils constitués par les nouvelles 2-thiazolyl imidazo [1,2-a] pyrimidines dont les noms suivent :
- la 6-éthyl-7-méthoxy-5-méthyl-2-(4-méthylthiazol-2-yl)imidazo [1,2-a] pyrimidine,
- la 6-éthyl-7-méthoxy-5-méthyl-2-(thiazol-2-yl)imidazo [1,2-a] pyrimidine,

ainsi que leurs sels d'addition avec les acides pharmaceutiquement acceptables.

10.- Compositions pharmaceutiques, caractérisées en ce qu'elles renferment à titre de principe actif, l'un au moins des médicaments tels que définis à l'une quelconque des revendications 7 à 9.

**Revendications pour l'Etat contractant suivant : AT**

1.- Procédé de préparation de nouvelles 2-thiazolyl imidazo [1,2-a] pyrimidines répondant à la formule (I) :

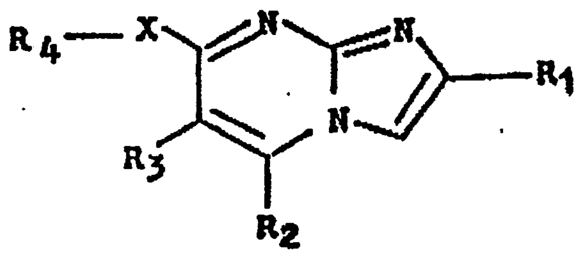

(I)

dans laquelle $R_1$ représente un radical 2-thiazolyl non substitué ou substitué soit par un ou deux radicaux alcoyles renfermant de1 à 3 atomes de carbone, ces radicaux pouvant être eux-mêmes non substitués ou substitués par un ou plusieurs atomes de fluor, soit par un groupe $-CO_2Alk$ dans lequel Alk représente un radical alcoyle renfermant de 1 à 3 atomes de carbone,
$R_2$ et $R_3$, identiques ou différents, représentent un atome d'hydrogène, un radical alcoyle renfermant de 1 à 3 atomes de carbone, un radical alcényle renfermant de 2 à 5 atomes de carbone, ou $R_2$ et $R_3$ forment ensemble un radical alcoylène linéaire renfermant de 3 à 5 atomes de carbone,
X représente un atome d'oxygène ou de soufre et $R_4$ représente un radical alcoyle renfermant de 1 à 3 atomes de carbone, ainsi que leurs sels d'addition avec les acides minéraux ou organiques, caractérisé en ce que l'on traite un produit de formule (II) :

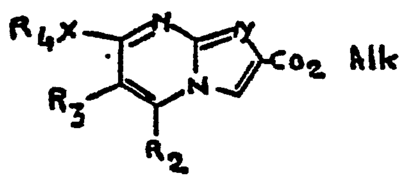

(II)

dans laquelle $R_2$, $R_3$, $R_4$ et X ont la signification déjà indiquée, par un agent basique puis acidifie pour obtenir un produit de formule (III) :

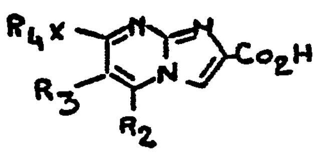

(III)

dans laquelle $R_2$, $R_3$, $R_4$ et X ont la signification déjà indiquée, produit de formule (III) que l'on traite par le N,N'-carbonyldiimidazole pour obtenir un produit de formule (IV) :

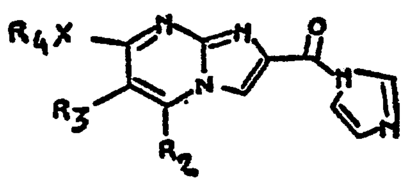

(IV)

dans laquelle $R_2$, $R_3$, $R_4$ et X ont la signification déjà indiquée, produit de formule (IV) que l'on traite par l'ammoniac, pour obtenir un produit de formule (V) :

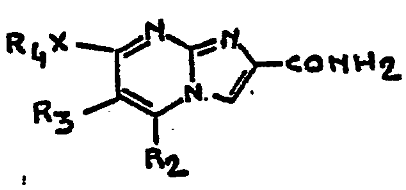

(V)

dans laquelle $R_2$, $R_3$, $R_4$ et X ont la signification déjà indiquée puis fait réagir le produit de formule (V) avec le réactif de Lawesson ou 2,4-bis-(4-méthoxyphényl)-1,3-dithia-2,4-diphosphétane-2,4-disulphide pour obtenir un produit de formule (VI) :

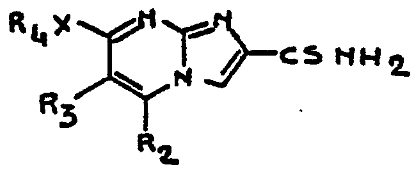

(VI)

dans laquelle $R_2$, $R_3$, $R_4$ et X ont la signification déjà indiquée, puis fait réagir le produit de formule (VI) avec un produit de formule (VII) :

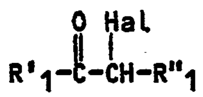

dans laquelle Hal représente un atome d'halogène, $R'_1$ et $R''_1$ identiques ou différents représentent soit un atome d'hydrogène, soit un radical alcoyle renfermant de 1 à 3 atomes de carbone non substitué ou substitué par un ou plusieurs atomes de fluor, soit par un radical $-CO_2Alk$ dans lequel Alk est défini comme ci-dessus, traite l'intermédiaire obtenu par un agent basique pour obtenir un produit de formule (I) que l'on isole et si désiré salifie.

2.- Procédé de préparation selon la revendication 7, caractérisé en ce que:
-l'agent basique que l'on fait réagir sur le produit de formule (II) est par exemple le carbonate de potassium et la réaction est effectuée au sein d'un mélange aqueux d'un solvant organique tel que le méthanol en chauffant à température de reflux du mélange réactionnel ;
- la réaction due produit de formule (III) avec le N,N'-carbonyldiimidazole est effectuée au sein d'un solvant organique, par exemple dans le diméthyl formamide ;
-le traitement à l'ammoniac du produit de formule (IV) est effectué dans un solvant organique tel que le chloroforme ;
- la réaction due produit de formule (V) avec le réactif de Lawesson est effectué au sein d'un solvant organique tel que le tétrahydrofuranne à température de reflux du milieu réactionnel ;
- dans la formule (VII) Hal représente de préférence un atome de chlore ;
- la réaction du produit de formule (VI) avec le produit de formule (VII) est effectuée au sein d'un solvant organique tel que l'éthanol et l'intermédiaire obtenu est soumis à un agent basique tel que l'ammoniaque concentrée.

3.- Procédé de préparation selon la revendication 1 ou 2, caractérisé en ce que l'on met en oeuvre au départ des produits choisis de manière telle que l'on prépare des produits de formule (I) dans laquelle $R_1$ représente un radical thiazol-2-yl, 4-méthylthiazol-2-yl, 4,5-diméthylthiazol-2-yl, 4-éthylthiazol-2-yl, 4-trifluorométhylthiazol-2-yl ou 4-éthoxycarbonyl-thiazol-2-yl.

4.- Procédé de préparation selon l'une quelconque des revendications 1 à 3, caractérisé en ce que l'on utilise au départ un produit de formule (II) dans laquelle $R_2$ et $R_3$, identiques ou différents, représentent un radical alcoyle renfermant 1 à 3 atomes de carbone ou un radical allyle ou $R_2$ et $R_3$ ensemble représentent le radical $(CH_2)_4$ et X représente un atome d'oxygène.

5.- Procédé de préparation selon l'une quelconque des revendications 1 à 4, caractérisé en ce que l'on prépare la 6-éthyl-7-méthoxy-5-méthyl-2-(4-méthylthiazol-2-yl)imidazo [1,2-a] pyrimidine et la 6-éthyl 7-méthoxy-5-méthyl 2-(thiazol-2-yl)imidazo [1,2-a] pyrimidine et leurs sels d'addition avec les acides.

6.- Procédé de préparation de compositions pharmaceutiques, caractérisés en ce que l'on met comme principe actif l'un au moins des produits de formule (I) tel qu'obtenu à la revendication 1 ou l'un au moins de ses sels pharmaceutiquement acceptables sous une forme adaptée à l'usage thérapeutique.

7.- Procédé de préparation de compositions pharmaceutiques, caractérisés en ce que l'on met comme principe actif l'un au moins des produits de formule (I) tel qu'obtenu à l'une quelconque des revendications 2 à 4 ou l'un au moins des ses sels pharmaceutiquement acceptables sous une forme adaptée à l'usage thérapeutique.

8.- Procédé de préparation de compositions pharmaceutiques, caractérisés en ce que l'on met comme principe actif l'un au moins des produits de formule (I) tel qu'obtenu à la revendication 5 ou l'un au moins de ses sels pharmaceutiquement acceptables sous une forme adaptée à l'usage thérapeutique.

**Revendications pour l'Etat contractant suivant : ES**

1.- Procédé de préparation de nouvelles 2-thiazolyl imidazo [1,2-a] pyrimidines répondant à la formule (I) :

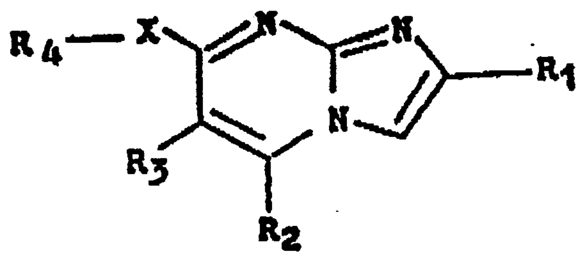

(I)

dans laquelle $R_1$ représente un radical 2-thiazolyl non substitué ou substitué soit par un ou deux radicaux alcoyles renfermant de1 à 3 atomes de carbone, ces radicaux pouvant être eux-mêmes non substitués ou substitués par un ou plusieurs atomes de fluor, soit par un groupe $-CO_2Alk$ dans lequel Alk représente un radical alcoyle renfermant de 1 à 3 atomes de carbone,
$R_2$ et $R_3$, identiques ou différents, représentent un atome d'hydrogène, un radical alcoyle renfermant de 1 à 3 atomes de carbone, un radical alcényle renfermant de 2 à 5 atomes de carbone, ou $R_2$ et $R_3$ forment ensemble un radical alcoylène linéaire renfermant de 3 à 5 atomes de carbone,
X représente un atome d'oxygène ou de soufre et $R_4$ représente un radical alcoyle renfermant de 1 à 3 atomes de carbone, ainsi que leurs sels d'addition avec les acides minéraux ou organiques, caractérisé en ce que l'on traite un produit de formule (II) :

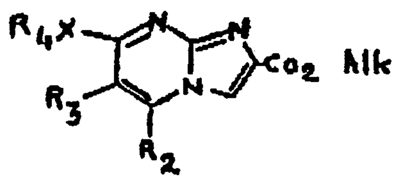

(II)

dans laquelle $R_2$, $R_3$, $R_4$ et X ont la signification déjà indiquée, par un agent basique puis acidifie pour obtenir un produit de formule (III) :

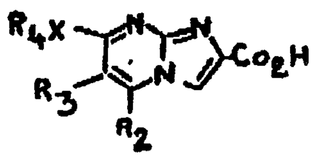

(III)

dans laquelle $R_2$, $R_3$, $R_4$ et X ont la signification déjà indiquée, produit de formule (III) que l'on traite par le N,N'-carbonyldiimidazole pour obtenir un produit de formule (IV) :

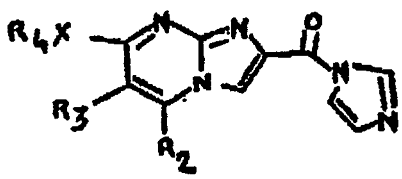

(IV)

dans laquelle $R_2$, $R_3$, $R_4$ et X ont la signification déjà indiquée, produit de formule (IV) que l'on traite par l'ammoniac, pour obtenir un produit de formule (V) :

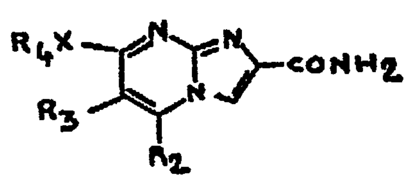

(V)

dans laquelle $R_2$, $R_3$, $R_4$ et X ont la signification déjà indiquée, puis fait réagir le produit de formule (V) avec le réactif de Lawesson ou 2,4-bis-(4-méthoxyphényl) 1,3-dithia 2,4-diphosphétane 2,4-disulphide pour obtenir unproduit de formule (VI) :

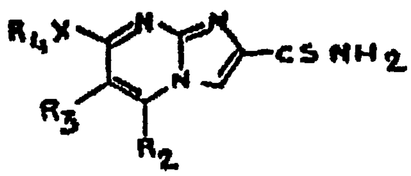

(VI)

dans laquelle $R_2$, $R_3$, $R_4$ et X ont la signification déjà indiquée, puis fait réagir le produit de formule (VI) avec un produit de formule (VII) :

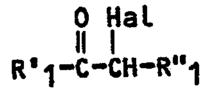

dans laquelle Hal représente un atome d'halogène, $R'_1$ et $R''_1$ identiques ou différents représentent soit un atome d'hydrogène, soit un radical alcoyle renfermant de 1 à 3 atomes de carbone non substitué ou substitué par un ou plusieurs atomes de fluor, soit par un radical $-CO_2Alk$ dans lequel Alk est défini comme ci-dessus, traite l'intermédiaire obtenu par un agent basique pour obtenir un produit de formule (I) que l'on isole et si désiré salifie.

2.- Procédé de préparation selon la revendication 7, caractérisé en ce que :

- l'agent basique que l'on fait réagir sur le produit de formule (II) est par exemple le carbonate de potassium et la réaction est effectuée au sein d'un mélange aqueux d'un solvant organique tel que le méthanol en chauffant à température de reflux du mélange réactionnel ;
- la réaction du produit de formule (III) avec le N,N'-carbonyldiimidazole est effectuée au sein d'un solvant organique, par exemple dans le diméthyl formamide ;
- le traitement à l'ammoniac du produit de formule (IV) est effectué dans un solvant organique tel que le chloroforme ;
- la réaction du produit de formule (V) avec le réactif de Lawesson est effectué au sein d'un solvant organique tel que le tétrahydrofuranne à température de reflux du milieu réactionnel ;
- dans la formule (VII) Hal représente de préférence un atome de chlore ;
- la réaction du produit de formule (VI) avec le produit de formule (VII) est effectuée au sein d'un solvant organique tel que l'éthanol et l'intermédiaire obtenu est soumis à un agent basique tel que l'ammoniaque concentrée.

3.- Procédé de préparation selon la revendication 1 ou 2, caractérisé en ce que l'on met en oeuvre au départ des produits choisis de manière telle que l'on prépare des produits de formule (I) dans laquelle $R_1$ représente un radical thiazol-2-yl, 4-méthylthiazol-2-yl, 4,5-diméthylthiazol-2-yl, 4-éthylthiazol-2-yl, 4-trifluorométhylthiazol-2-yl ou 4-éthoxycarbonylthiazol-2-yl.

4.- Procéde de préparation selon l'une quelconque des revendications 1 à 3, caractérisé en ce que l'on utilise au départ un produit de formule (II) dans laquelle $R_2$ et $R_3$, identiques ou différents, représentent un radical alcoyle renfermant 1 à 3 atomes de carbone ou un radical allyle ou $R_2$ et $R_3$ ensemble représentent le radical $(CH_2)_4$ et X représente un atome d'oxygène.

**Revendications pour l'Etat contractant suivant : GR**

1.- Procédé de préparation de nouvelles 2-thiazolyl imidazo [1,2-a] pyrimidines répondant à la formule (I) :

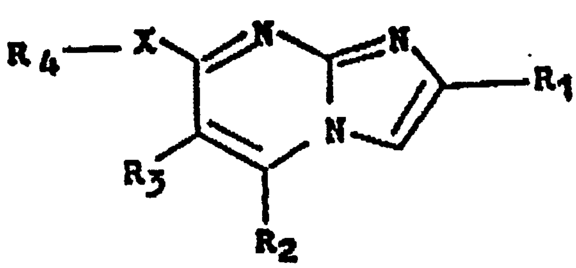

(I)

dans laquelle $R_1$ représente un radical 2-thiazolyl non substitué ou substitué soit par un ou deux radicaux alcoyles renfermant de1 à 3 atomes de carbone, ces radicaux pouvant être eux-mêmes non substitués ou substitués par un ou plusieurs atomes de fluor, soit par un groupe $-CO_2Alk$ dans lequel Alk représente un radical alcoyle renfermant de 1 à 3 atomes de carbone,
$R_2$ et $R_3$, identiques ou différents, représentent un atome d'hydrogène, un radical alcoyle renfermant de 1 ä 3 atomes de carbone, un radical alcényle renfermant de 2 à 5 atomes de carbone, ou $R_2$ et $R_3$ forment ensemble un radical acoylène linéaire renfermant de 3 à 5 atomes de carbone,
X représente un atome d'oxygène ou de soufre et $R_4$ représente un radical alcoyle renfermant de 1 à 3 atomes de carbone, ainsi que leurs sels d'addition avec les acides minéraux ou organiques, caractérisé

en ce que l'on traite un produit de formule (II) :

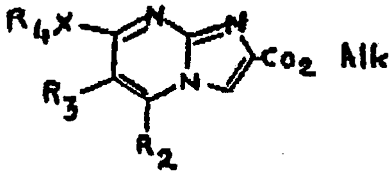

(II)

dans laquelle $R_2$, $R_3$, $R_4$ et X ont la signification déjà indiquée, par un agent basique puis acidifie pour obtenir un produit de formule (III) :

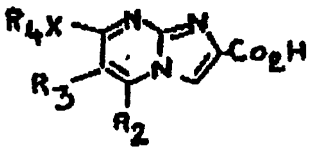

(III)

dans laquelle $R_2$, $R_3$, $R_4$ et X ont la signification déjà indiquée, produit de formule (III) que l'on traite par le N,N'-carbonyldiimidazole pour obtenir un produit de formule (IV) :

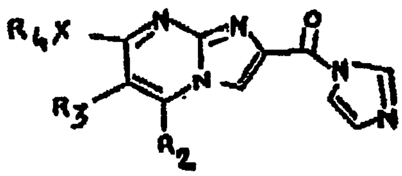

(IV)

dans laquelle $R_2$, $R_3$, $R_4$ et X ont la signification déjà indiquée, produit de formule (IV) que l'on traite par l'ammoniac, pour obtenir un produit de formule (V) :

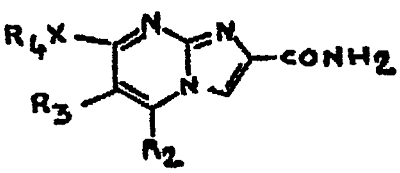

(V)

dans laquelle $R_2$, $R_3$, $R_4$ et X ont la signification déjà indiquée puis fait réagir le produit de formule (V) avec le réactif de Lawesson ou 2,4-bis-(4-méthoxyphényl) 1,3-dithia 2,4-diphosphétane 2,4-disulphide pour obtenir un produit de formule (VI) :

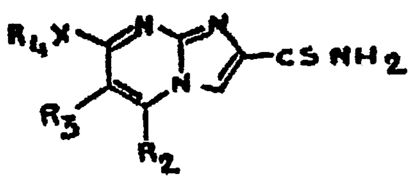

(VI)

dans laquelle $R_2$, $R_3$, $R_4$ et X ont la signification déjà indiquée, puis fait réagir le produit de formule (VI) avec un produit de formule (VII) :

$$R'_1-\overset{\overset{O}{\|}}{C}-\overset{\overset{Hal}{|}}{CH}-R''_1$$

dans laquelle Hal représente un atome d'halogène, $R'_1$ et $R''_1$ identiques ou différents représentent soit un atome d'hydrogène, soit un radical alcoyle renfermant de 1 à 3 atomes de carbone non substitué ou substitué par un ou plusieurs atomes de fluor, soit par un radical $-CO_2Alk$ dans lequel Alk est défini comme ci-dessus, traite l'intermédiaire obtenu par un agent basique pour obtenir un produit de formule (I) que l'on isole et si désiré salifie.

2.- Procédé de préparation selon la revendication 7, caractérisé en ce que :
- l'agent basique que l'on fait réagir sur le produit de formule (II) est par exemple le carbonate de potassium et la réaction est effectuée au sein d'un mélange aqueux d'un solvant organique tel que le méthanol en chauffant à température de reflux du mélange réactionnel ;

- la réaction du produit de formule (III) avec le N,N'-carbonyldiimidazole est effectuée au sein d'un solvant organique, par exemple dans le diméthyl formamide ;
- le traitement à l'ammoniac du produit de formule (IV) est effectué dans un slovant organique tel que le chloroforme ;
- la réaction du produit de formule (V) avec le réactif de Lawesson est effectué au sein d'un solvant organique tel que le tétrahydrofuranne à température de reflux du milieu réactionnel ;
- dans la formule (VII)n Hal représente de préférence un atome de chlore ;
- la réaction du produit de formule (VI) avec le produit de formule (VII) est effectuée au sein d'un solvant organique tel que l'éthanol et l'intermédiaire obtenu est soumis à un agent basique tel que l'ammoniaque concentrée.

3.- Procédé de préparation selon la revendication 1 ou 2, caractérisé en ce que l'on met en oeuvre au départ des produits choisis de manière telle que l'on prépare des produits de formule (I) dans laquelle $R_1$ représente un radical thiazol-2-yl, 4-méthylthiazol-2-yl, 4,5-diméthylthiazol-2-yl, 4-éthylthiazol-2-yl, 4-trifluorométhylthiazol-2-yl ou 4-éthoxycarbonylthiazol-2-yl.

4.- Procédé de préparation selon l'une quelconque des revendications 1 à 3, caractérisé en ce que l'on utilise au départ un produit de formule (II) dans laquelle $R_2$ et $R_3$, identiques ou différents, représentent un radical alcoyle renfermant 1 à 3 atomes de carbone ou un radical allyle ou $R_2$ et $R_3$ ensemble représentent le radical $(CH_2)_4$ et X représente un atome d'oxygène.

5.- Procédé de préparation selon l'une quelconque des revendications 1 à 4, caractérisé en ce que l'on prépare la 6-éthyl-7-méthoxy-5-méthyl-2-(4-méthylthiazol-2-yl)imidazo [1,2-a] pyrimidine et la 6-éthyl-7-méthoxy-5-méthyl-2-(thiazol-2-yl)imidazo [1,2-a] pyrimidine et leurs sels d'addition avec les acides.

6.- Procédé de préparation de compositions pharmaceutiques, caractérisés en ce que l'on met comme principe actif l'un au moins des produits de formule (I) tel qu'obtenu à la revendication 1 ou l'un au moins de ses sels pharmaceutiquement acceptables sous une forme adaptée à l'usage thérapeutique.

7.- Procédé de préparation de compositons pharmaceutiques, caractérisés en ce que l'on met comme principe actif l'un au moins des produits de formule (I) tel qu'obtenu à l'une quelconque des revendications 2 à 4 ou l'un au moins de ses sels pharmaceutiquement acceptables sous une forme adaptée à l'usage thérapeutique.

8.- Procédé de préparation de compositions pharmaceutiques, caractérisés en ce que l'on met comme principe actif l'un au moins des produits de formule (I) tel qu'obtenu à la revendication 5 ou l'un au moins de ses sels pharmaceutiquement acceptables sous une forme adaptée à l'usage thérapeutique.

**Patentansprüche für die Vertragsstaaten: BE, CH, DE, FR, IT, LI, LU, NL, SE**

1. Neue 2-Thiazolylimidazo[1,2-a]pyrimidine der Formel (I)

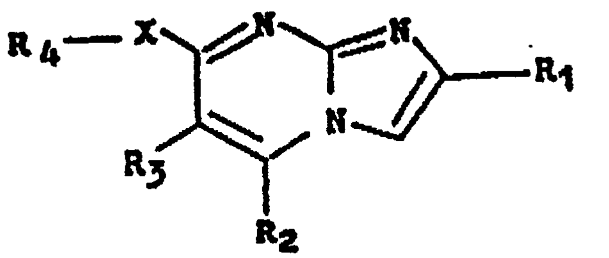

(I)

worin $R_1$ für einen 2-Thiazolylrest steht, der nicht substituiert ist durch einen oder zwei Alkylreste mit 1 bis 3 Kohlenstoffatomen, wobei diese Reste ihrerseits nicht substituiert sind oder substituiert sind durch ein oder mehrere Fluoratome oder durch eine Gruppe $-CO_2Alk$, worin Alk für einen Alkylrest mit 1 bis 3 Kohlenstoffatomen steht, $R_2$ und $R_3$, die gleich oder voneinander verschieden sind, für ein Wasserstoffatom, einen Alkylrest mit 1 bis 3 Kohlenstoffatomen, einen Alkenylrest mit 2 bis 5 Kohlenstoffatomen stehen oder $R_2$ und $R_3$ gemeinsam einen linearen Alkylenrest mit 3 bis 5 Kohlenstoffatomen bilden,
X für ein Sauerstoff- oder Schwefelatom steht und
$R_4$ für eine Alkylgruppe mit 1 bis 3 Kohlenstoffatomen steht,
sowie deren Additionssalze mit Mineral- oder organischen Säuren

2. Neue 2-Thiazolylimidazo[1,2-a]pyrimidine gemäß Anspruch 1 und deren Additionssalze mit Säuren, dadurch gekennzeichnet, daß in der Formel (I) $R_1$ für einen Thiazol-2-yl, 4-Methylthiazol-2-yl-, 4,5-Dimethylthiazol-2-yl-, 4-Ethylthiazol-2-yl, 4-Trifluormethylthiazol-2-yl- oder 4-Ethoxycarbonylthiazol-2-yl-Rest steht.

3. Neue 2-Thiazolylimidazo[1,2-a]pyrimidine gemäß Anspruch 1 oder 2 und deren Additionssalze mit Säuren, dadurch gekennzeichnet, daß in der Formel (I) $R_2$ und $R_3$, die gleich oder voneinander verschieden sind, für einen Alkylrest mit 1 bis 3 Kohlenstoffatomen oder einen Allylrest stehen oder $R_2$ und $R_3$ gemeinsam den Rest $(CH_2)_4$ wiedergeben und X ein Sauerstoffatom bedeutet.

4. Eines der neuen 2-Thiazolylimidazo[1,2-a]pyrimidine der Formel (I) gemäß Anspruch 1 mit den folgenden Bezeichnungen:
6-Ethyl-7-methoxy-5-methyl-2-(4-methylthiazol-2-yl)-imidazo[1,2-a]pyrimidin
6-Ethyl-7-methoxy-5-methyl-2-(thiazol-2-yl)-imidazo[1,2-a]pyrimidin
sowie deren Additionssalze mit Säuren.

5. Verfahren zur Herstellung der neuen 2-Thiazolylimidazo[1,2-a]pyrimidine der Formel (I) gemäß Anspruch 1 sowie von deren Additionssalzen mit Säuren, dadurch gekennzeichnet, daß man ein Produkt der Formel (II)

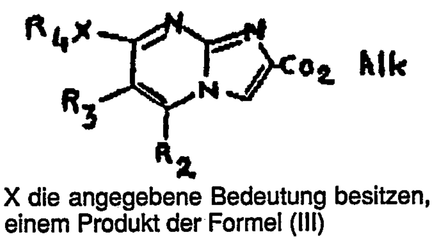

(II)

worin $R_2$, $R_3$, $R_4$ und X die angegebene Bedeutung besitzen, mit einem basischen Mittel behandelt, hiernach ansäuert, um zu einem Produkt der Formel (III)

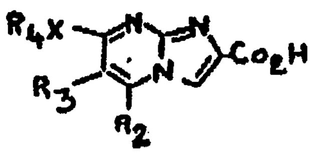

(III)

zu gelangen, worin $R_2$, $R_3$, $R_4$ und X die angegebene Bedeutung besitzen, das Produkt der Formel (III) mit N,N'-Carbonyldiimidazol behandelt, um zu einem Produkt der Formel (IV)

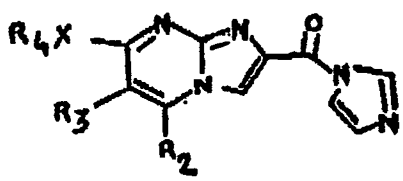

(IV)

zu gelangen, worin $R_2$, $R_3$, $R_4$ und X die angegebene Bedeutung besitzen, das Produkt der Formel (IV) mit Ammoniak behandelt, um zu einem Produkt der Formel (V)

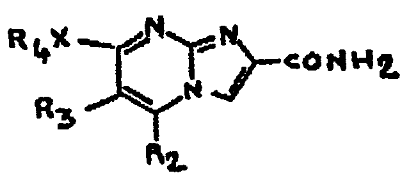

(V)

zu gelangen, worin $R_2$, $R_3$, $R_4$ und X die angegebene Bedeutung besitzen, hiernach das Produkt der Formel (V) mit dem Lawesson-Reagens oder 2,4-Bis-(4-methoxyphenyl)-1,3-dithia-2,4-diphosphetan-2,4-disulfid umsetzt, um zu einem Produkt der Formel (VI)

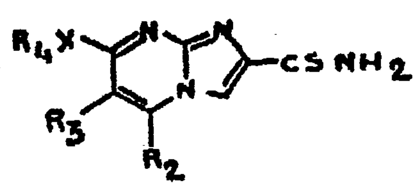

(VI)

zu gelangen, worin $R_2$, $R_3$, $R_4$ und X die angegebene Bedeutung besitzen, hiernach das Produkt der Formel (VI) mit einem Produkt der Formel (VII)

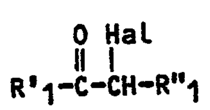

umsetzt, worin Hal für ein Halogenatom steht, R'1 und R"1, die gleich oder voneinander verschieden sind, entweder ein Wasserstoffatom oder einen Alkylrest mit 1 bis 3 Kohlenstoffatomen, der nicht substituiert ist oder substituiert ist durch ein oder mehrere Fluoratome oder durch einen Rest $-CO_2Alk$, worin

Alk wie vorstehend definiert ist, bedeuten, das erhaltene Zwischenprodukt mit einem basischen Mittel behandelt, um zu einem Produkt der Formel (I) zu gelangen, das man isoliert und gewünschtenfalls in ein Salz überführt.

6. Verfahren gemäß Anspruch 5, dadurch gekennzeichnet, daß das basische Mittel, das man mit dem Produkt der Formel (II) umsetzt, z.B. Kaliumcarbonat ist und die Umsetzung in einem wäßrigen Gemisch eines organischen Lösungsmittels, wie Methanol, durchgeführt wird, indem man auf die Rückflußtemperatur des Reaktionsgemisches erhitzt; die Reaktion des Produkts der Formel (III) mit N,N'-Carbonyldiimidazol in einem organischen Lösungsmittel, z.B. Dimethylformamid, durchgeführt wird; die Behandlung mit Ammoniak des Produkts der Formel (IV) in einem organischen Lösungsmittel, wie Chloroform, durchgeführt wird; die Umsetzung des Produkts der Formel (VI) mit dem Lawesson-Reagens in einem organischen Lösungsmittel, wie Tetrahydrofuran, bei der Rückflußtemperatur des Reaktionsmilieus durchgeführt wird; in der Formel (VII) Hal vorzugsweise ein Chloratom bedeutet; die Umsetzung des Produkts der Formel (VI) mit dem Produkt der Formel (VII) in einem organischen Lösungsmittel, wie Ethanol, durchgeführt wird und das erhaltene Zwischenprodukt einem basischen Mittel, wie konzentriertem Ammoniak, ausgesetzt wird.

7. Arzneimittel, dadurch gekennzeichnet, daß sie aus den neuen 2-Thiazolylimidazo[1,2-a]pyrimidinen der Formel (I) gemäß Anspruch 1 sowie aus deren Additionssalzen mit pharmazeutisch verträglichen Säuren bestehen.

8. Arzneimittel, dadurch gekennzeichnet, daß sie aus den neuen 2-Thiazolylimidazo[1,2-a]pyrimidinen gemäß einem der Ansprüche 2 oder 3 sowie aus deren Additionssalzen mit pharmazeutisch verträglichen Säuren bestehen.

9. Arzneimittel, dadurch gekennzeichnet, daß sie aus den neuen 2-Thiazolylimidazo[1,2-a]pyrimidinen mit den folgenden Bezeichnungen
6-Ethyl-7-methoxy-5-methyl-2-(4-methylthiazol-2-yl)-imidazo[1,2-a]pyrimidin,
6-Ethyl-7-methoxy-5-methyl-2-(thiazol-2-yl)-imidazo[1,2-a]pyrimidin
sowie aus deren Additionssalzen mit pharmazeutisch verträglichen Säuren bestehen.

10. Pharmazeutische Zusammensetzungen, dadurch gekennzeichnet, daß sie als Wirkstoff zumindest eines der Arzneimittel gemäß einem der Ansprüche 7 bis 9 enthalten.

**Patentansprüche für den Vertragstaat: AT**

1. Verfahren zur Herstellng von neuen 2-Thiaazolylimidazo[1,2-a]pyrimidinen der Formel (I)

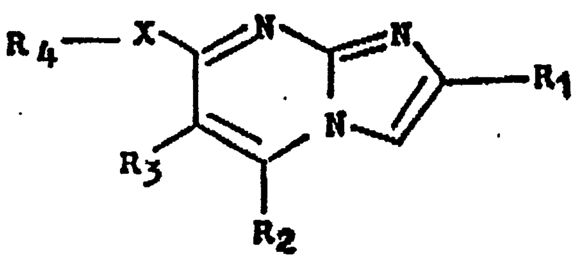

(I)

worin $R_1$ für einen 2-Thiazolylrest steht, der nicht substituiert ist oder substituiert ist durch einen oder zwei Alkylreste mit 1 bis 3 Kohlenstoffatomen, wobei diese Reste ihrerseits nicht substituiert sind oder substituiert sind durch ein oder mehrere Fluoratome oder durch eine Gruppe $-CO_2Alk$, worin Alk für einen Alkylrest mit 1 bis 3 Kohlenstoffatomen steht, $R_2$ und $R_3$, die gleich oder voneinander verschieden sind, für ein Wasserstoffatom, einen Alkylrest mit 1 bis 3 Kohlenstoffatomen, einen Alkenylrest mit 2 bis 5 Kohlenstoffatomen stehen oder $R_2$ und $R_3$ gemeinsam einen linearen Alkylenrest mit 3 bis 5 Kohlenstoffatomen bilden
X für Sauerstoff- oder Schwefelatom steht und
$R_4$ für eine Alkylgruppe mit 1 bis 3 Kohlenstoffatomen steht,
sowie ihrer Additionssalze mit Mineral- oder organischen Säuren, dadurch gekennzeichnet, daß man ein Produkt der Formel (II)

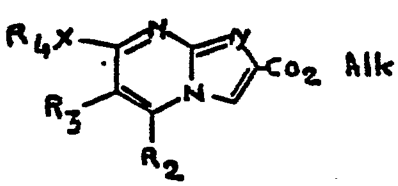

(II)

worin $R_2$, $R_3$, $R_4$ und X die angegebene Bedeutung besitzen, mit einem basischen Mittel behandelt, hiernach ansäuert, um zu einem Produkt der Formel (III)

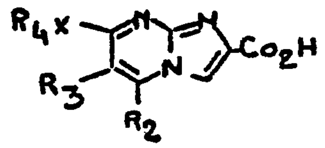

(III)

zu gelangen, worin $R_2$, $R_3$, $R_4$ und X die angegebene Bedeutung besitzen, das Produkt der Formel (III) mit N, N'-Carbonyldiimidazol behandelt, um zu einem Produkt der Formel (IV)

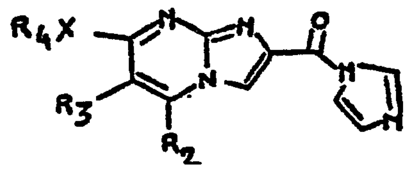

(IV)·

zu gelangen, worin $R_2$, $R_3$, $R_4$ und X die angegebene Bedeutung besitzen, das Produkt der Formel (IV) mit Ammoniak behandelt, um zu einem Produkt der Formel (V)

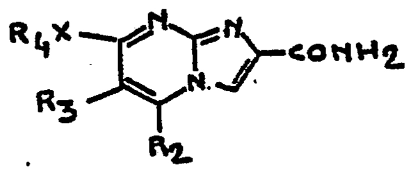

(V)

zu gelangen, worin $R_2$, $R_3$, $R_4$ und X die angegebene Bedeutung besitzen, hiernach das Produkt der Formel (V) mit dem Lawesson-Reagens oder 2,4-Bis-(4-methoxyphenyl)-1,3-dithia-2,4-diphosphetan-2,4-disulfid umsetzt, um zu einem Produkt der Formel (VI)

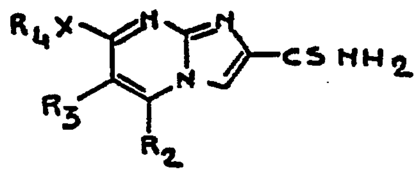

(VI)

zu gelangen, worin $R_2$, $R_3$, $R_4$ und X die angegebene Bedeutung besitzen, hiernach das Produkt der Formel (VI) mit einem Produkt der Formel (VII)

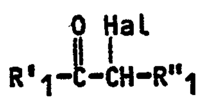

umsetzt, worin Hal für ein Halogenatom steht, R'1 und R"1, die gleich oder voneinander verschieden sind, entweder ein Wasserstoffatom oder einen Alkylrest mit 1 bis 3 Kohlenstoffatomen, der nicht substituiert ist oder substituiert ist durch ein oder mehrere Fluoratome oder durch einen Rest $-CO_2Alk$, worin Alk wie vorstehend definiert ist, bedeuten, das erhaltene Zwischenprodukt mit einem basischen Mittel behandelt, um zu einem Produkt der Formel (I) zu gelangen, das man isoliert und gewünschtenfalls in ein Salz überführt.

2. Verfahren gemäß Anspruch 1, dadurch gekennzeichnet, daß das basische Mittel, das man mit dem Produkt der Formel (II) umsetzt, z.B. Kaliumcarbonat ist und die Umsetzung in einem wäßrigen Gemisch eines organischen Lösungsmittels, wie Methanol, durchgeführt wird, indem man auf die Rückflußtemperatur des Reaktionsgemisches erhitzt; die Reaktion des Produkts der Formel (III) mit N,N'-Carbonyldiimidazol in einem organischen Lösungsmittel, z.B. Dimethylformamid, durchgeführt wird; die Behandlung mit Ammoniak des Produkts der Formel (IV) in einem organischen Lösungsmittel, wie Chloroform, durchgeführt wird; die Umsetzung des Produkts der Formel (V) mit dem Lawesson-Reagens in einem organischen Lösungsmittel, wie Tetrahydrofuran, bei der Rückflußtemperatur des Reaktionsmilieus durchgeführt wird; in der Formel (VII) Hal vorzugsweise ein Chloratom bedeutet; die Umsetzung des Produkts der Formel (VI) mit dem Produkt der Formel (VII) in einem organischen Lösungsmittel, wie Ethanol, durchgeführt wird und das erhaltene Zwischenprodukt einem basischen Mittel, wie konzentriertem Ammoniak, ausgesetzt wird.

3. Verfahren gemäß Anspruch 1 oder 2, dadurch gekennzeichnet, daß man von Produkten ausgeht, die derart ausgewählt sind, daß man die Produkte der Formel (I) herstellt, worin $R_1$ einen Thiazol-2-yl-, 4-Methylthiazol-2-yl-, 4,5-Dimethylthiazol-2-yl-, 4-Ethylthiazol-2-yl-, 4-Trifluormethylthiazol-2-yl- oder 4-Ethoxycarbonylthiazol-2-yl-Rest bedeutet.

4. Verfahren gemäß einem der Ansprüche 1 bis 3, dadurch gekennzeichnet, daß man von einem Produkt der Formel (II) ausgeht, worin $R_2$ und $R_3$, die gleich oder voneinander verschieden sind, für einen Alkylrest mit 1 bis 3 Kohlenstoffatomen oder einen Allylrest stehen oder $R_2$ und $R_3$ gemeinsam den Rest $(CH_2)_4$ bilden und X ein Sauerstoffatom bedeutet.

5. Verfahren gemäß einem der Ansprüche 1 bis 4, dadurch gekennzeichnet, daß man 6-Ethyl-7-methoxy-5-methyl-2-(4-methylthiazol-2-yl)-imdazo[1,2-a]pyrimidin und 6-Ethyl-7-methoxy-5-methyl-2-(thiazol-2-yl)-imidazo[1,2-a]pyrimidin und deren Additionssalze mit Säuren herstellt.

6. Verfahren zur Herstellung pharmazeutischer Zusammensetzungen, dadurch gekennzeichnet, daß man als Wirkstoff zumindest eines der Produkte der Formel (I), erhalten gemäß Anspruch 1, oder zumindest eines ihrer pharmazeutisch verträglichen Salze in eine für die therapeutische Verwendung geeignete Form überführt.

7. Verfahren zur Herstellung pharmazeutischer Zusammensetzungen, dadurch gekennzeichnet, daß man als Wirkstoff zumindest eines der Produkte der Formel (I), erhalten gemäß einem der Ansprüche 2 bis 4, oder zumindest eines ihrer pharmazeutisch verträglichen Salze in eine für die therapeutische Verwendung geeignete Form überführt.

8. Verfahren zur Herstellung pharmazeutischer Zusammensetzungen, dadurch gekennzeichnet, daß man als Wirkstoff zumindest eines der Produkte der Formel (I), erhalten gemäß Anspruch 5, oder zumindest eines ihrer pharmazeutisch verträglichen Salze in eine für die therapeutische Verwendung geeignete Form überführt.

**Patentansprüche für den Vertragsstaat: ES**

1. Verfahren zur Herstellng von neuen 2-Thiazolylimidazo[1,2-a]pyrimidinen der Formel

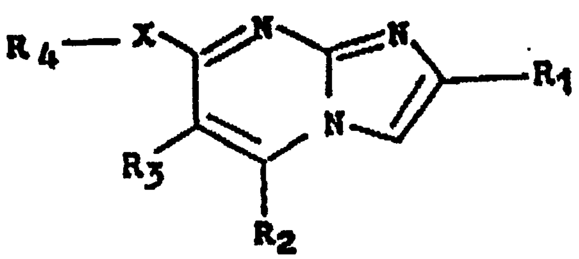

(I)

worin $R_1$ für einen 2-Thiazolylrest steht, der nicht substituiert ist oder substituiert ist durch einen oder zwei Alkylreste mit 1 bis 3 Kohlenstoffatomen, wobei diese Reste ihrerseits nicht substituiert sind oder substituiert sind durch ein oder mehrere Fluoratome oder durch eine Gruppe $-CO_2Alk$, worin Alk für einen Alkylrest mit 1 bis 3 Kohlenstoffatomen steht, $R_2$ und $R_3$, die gleich oder voneinander verschieden sind, für ein Wasserstoffatom, einen Alkylrest mit 1 bis 3 Kohlenstoffatomen, einen Alkenylrest mit 2 bis 5 Kohlenstoffatomen stehen oder $R_2$ und $R_3$ gemeinsam einen linearen Alkylenrest mit 3 bis 5 Kohlenstoffatomen bilden
X für Sauerstoff- oder Schwefelatom steht und
$R_4$ für eine Alkylgruppe mit 1 bis 3 Kohlenstoffatomen steht,

sowie ihrer Additionssalze mit Mineral- oder organischen Säuren, dadurch gekennzeichnet, daß man ein Produkt der Formel (II)

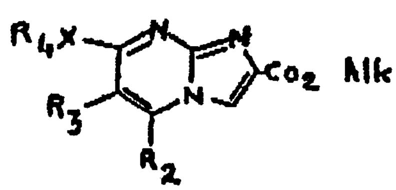

(II)

worin $R_2$, $R_3$, $R_4$ und X die angegebene Bedeutung besitzen, mit einem basischen Mittel behandelt, hiernach ansäuert, um zu einem Produkt der Formel (III)

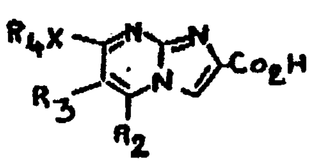

(III)

zu gelangen, worin $R_2$, $R_3$, $R_4$ und X die angegebene Bedeutung besitzen, das Produkt der Formel (III) mit N, N'-Carbonyldiimidazol behandelt, um zu einem Produkt der Formel (IV)

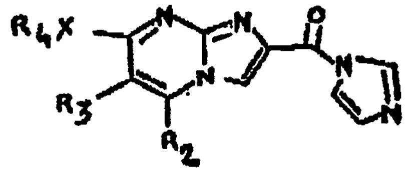

(IV)

zu gelangen, worin $R_2$, $R_3$, $R_4$ und X die angegebene Bedeutung besitzen, das Produkt der Formel (IV) mit Ammoniak behandelt, um zu einem Produkt der Formel (V)

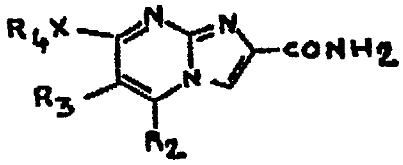

(V)

zu gelangen, worin $R_2$, $R_3$, $R_4$ und X die angegebene Bedeutung besitzen, hiernach das Produkt der Formel (V) mit dem Lawesson-Reagens oder 2,4-Bis-(4-methoxyphenyl)-1,3-dithia-2,4-diphosphetan-2,4-disulfid umsetzt, um zu einem Produkt der Formel (VI)

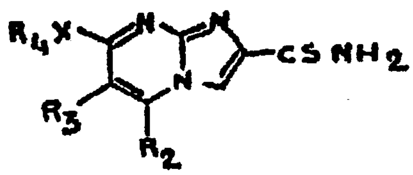

(VI)

zu gelangen, worin $R_2$, $R_3$, $R_4$ und X die angegebene Bedeutung besitzen, hiernach das Produkt der Formel (VI) mit einem Produkt der Formel (VII)

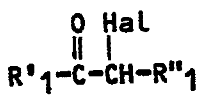

umsetzt, worin Hal für ein Halogenatom steht, R'1 und R"1, die gleich oder voneinander verschieden sind, entweder ein Wasserstoffatom oder einen Alkylrest mit 1 bis 3 Kohlenstoffatomen, der nicht substituiert ist oder substituiert ist durch ein oder mehrere Fluoratome oder durch einen Rest $-CO_2Alk$, worin Alk wie vorstehend definiert ist, bedeuten, das erhaltene Zwischenprodukt mit einem basischen Mittel behandelt, um zu einem Produkt der Formel (I) zu gelangen, das man isoliert und gewünschtenfalls in ein Salz überführt.

2. Verfahren gemäß Anspruch 1, dadurch gekennzeichnet, daß das basische Mittel, das man mit dem Produkt der Formel (II) umsetzt, z.B. Kaliumcarbonat ist und die Umsetzung in einem wäßrigen Gemisch eines organischen Lösungsmittels, wie Methanol, durchgeführt wird, indem man auf die Rückflußtemperatur des Reaktionsgemisches erhitzt; die Reaktion des Produkts der Formel (III) mit N,N'-Carbonyldiimidazol in einem organischen Lösungsmittel, z.B. Dimethylformamid, durchgeführt wird; die Behandlung mit Ammoniak des Produkts der Formel (IV) in einem organischen Lösungsmittel, wie Chloroform, durchgeführt wird; die Umsetzung des Produkts der Formel (V) mit dem Lawesson-Reagens in einem organischen Lösungsmittel, wie Tetrahydrofuran, bei der Rückflußtemperatur des Reaktionsmilieus durchgeführt wird; in der Formel (VII) Hal vorzugsweise ein Chloratom bedeutet; die Umsetzung des Produkts der Formel (VI) mit dem Produkt der Formel (VII) in einem organischen Lösungsmittel, wie Ethanol, durchgeführt wird und das erhaltene Zwischenprodukt einem basischen Mittel, wie konzentriertem Ammoniak, ausgesetzt wird.

3. Verfahren gemäß Anspruch 1 oder 2, dadurch gekennzeichnet, daß man von Produkten ausgeht, die derart ausgewählt sind, daß man die Produkte der Formel (I) herstellt, worin $R_1$ einen Thiazol-2-yl-, 4-Methylthiazol-2-yl-, 4,5-Dimethylthiazol-2-yl-, 4-Ethylthiazol-2-yl-, 4-Trifluormethylthiazol-2-yl- oder 4-Ethoxycarbonylthiazol-2-yl-Rest bedeutet.

4. Verfahren gemäß einem der Ansprüche 1 bis 3, dadurch gekennzeichnet, daß man von einem Produkt der Formel (II) ausgeht, worin $R_2$ und $R_3$, die gleich oder voneinander verschieden sind, für einen Alkylrest mit 1 bis 3 Kohlenstoffatomen oder einen Allylrest stehen oder $R_2$ und $R_3$ gemeinsam den Rest $(CH_2)_4$ bilden und X ein Sauerstoffatom bedeutet.

**Patentansprüche für den Vertragsstaat: GR**

1. Verfahren zur Herstellng von neuen 2-Thiazolylimidazo[1,2-a]pyrimidinen der Formel (I)

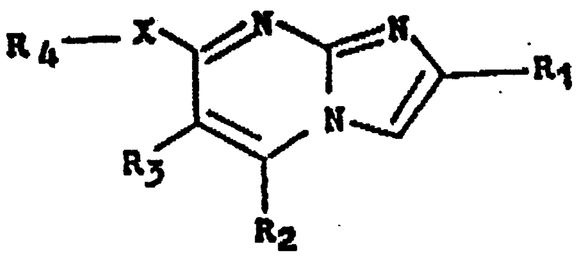

(I)

worin $R_1$ für einen 2-Thiazolylrest steht, der nicht substituiert ist oder substituiert ist durch einen oder zwei Alkylreste mit 1 bis 3 Kohlenstoffatomen, wobei diese Reste ihrerseits nicht substituiert sind oder substituiert sind durch ein oder mehrere Fluoratome oder durch eine Gruppe $-CO_2Alk$, worin Alk für einen Alkylrest mit 1 bis 3 Kohlenstoffatomen steht, $R_2$ und $R_3$, die gleich oder voneinander verschieden sind, für ein Wasserstoffatom, einen Alkylrest mit 1 bis 3 Kohlenstoffatomen, einen Alkenylrest mit 2 bis 5 Kohlenstoffatomen stehen oder $R_2$ und $R_3$ gemeinsam einen linearen Alkylenrest mit 3 bis 5 Kohlenstoffatomen bilden

X für Sauerstoff- oder Schwefelatom steht und

$R_4$ für eine Alkylgruppe mit 1 bis 3 Kohlenstoffatomen steht,

sowie ihrer Additionssalze mit Mineral- oder organischen Säuren, dadurch gekennzeichnet, daß man ein Produkt der Formel (II)

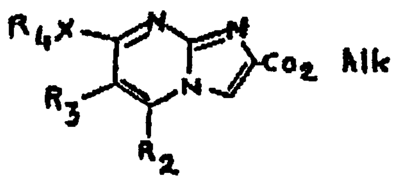

(II)

worin $R_2$, $R_3$, $R_4$ und X die angegebene Bedeutung besitzen, mit einem basischen Mittel behandelt, hiernach ansäuert, um zu einem Produkt der Formel (III)

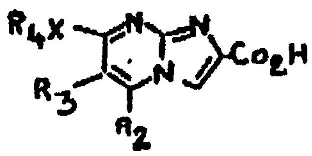

(III)

zu gelangen, worin $R_2$, $R_3$, $R_4$ und X die angegebene Bedeutung besitzen, das Produkt der Formel (III) mit N, N'-Carbonyldiimidazol behandelt, um zu einem Produkt der Formel (IV)

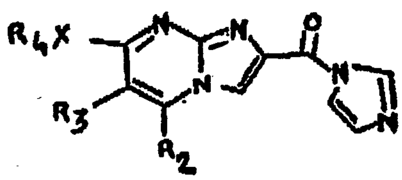

(IV)

zu gelangen, worin $R_2$, $R_3$, $R_4$ und X die angegebene Bedeutung besitzen, das Produkt der Formel (IV) mit Ammoniak behandelt, um zu einem Produkt der Formel (V)

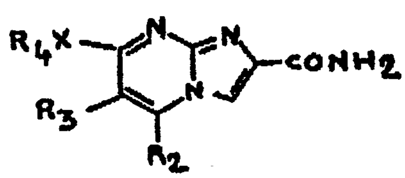

(V)

zu gelangen, worin $R_2$, $R_3$, $R_4$ und X die angegebene Bedeutung besitzen, hiernach das Produkt der Formel (V) mit dem Lawesson-Reagens oder 2,4-Bis-(4-methoxyphenyl)-1,3-dithia-2,4-diphosphetan-2,4-disulfid umsetzt, um zu einem Produkt der Formel (VI)

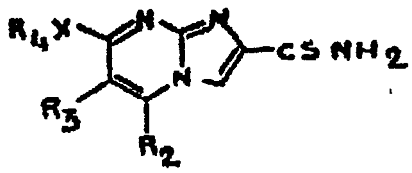

(VI)

zu gelangen, worin $R_2$, $R_3$, $R_4$ und X die angegebene Bedeutung besitzen, hiernach das Produkt der Formel (VI) mit einem Produkt der Formel (VII)

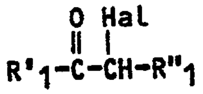

umsetzt, worin Hal für ein Halogenatom steht, R'1 und R"1, die gleich oder voneinander verschieden sind, entweder ein Wasserstoffatom oder einen Alkylrest mit 1 bis 3 Kohlenstoffatomen, der nicht substituiert ist oder substituiert ist durch ein oder mehrere Fluoratome oder durch einen Rest $-CO_2Alk$, worin Alk wie vorstehend definiert ist, bedeuten, das erhaltene Zwischenprodukt mit einem basischen Mittel behandelt, um zu einem Produkt der Formel (I) zu gelangen, das man isoliert und gewünschtenfalls in ein Salz überführt.

2. Verfahren gemäß Anspruoh 1, dadurch gekennzeichnet, daß das basische Mittel, das man mit dem Produkt der Formel (II) umsetzt, z.B. Kaliumcarbonat ist und die Umsetzung in einem wäßrigen Gemisch eines organischen Lösungsmittels, wie Methanol, durchgeführt wird, indem man auf die Rückflußtemperatur des Reaktionsgemisches erhitzt; die Reaktion des Produkts der Formel (III) mit N,N'-Carbonyldiimidazol in einem organischen Lösungsmittel, z.B. Dimethylformamid, durchgeführt wird; die Behandlung mit Ammoniak des Produkts der Formel (IV) in einem organischen Lösungsmittel, wie Chloroform, durchgeführt wird; die Umsetzung des Produkts der Formel (V) mit dem Lawesson-Reagens in einem organischen Lösungsmittel, wie Tetrahydrofuran, bei der Rückflußtemperatur des Reaktionsmilieus durchgeführt wird; in der Formel (VII) Hal vorzugsweise ein Chloratom bedeutet; die Umsetzung des Produkts der Formel (VI) mit dem Produkt der Formel (VII) in einem organischen Lösungsmittel, wie Ethanol, durchgeführt wird und das erhaltene Zwischenprodukt einem basischen Mittel, wie konzentriertem Ammoniak, ausgesetzt wird.

3. Verfahren gemäß Anspruch 1 oder 2, dadurch gekennzeichnet, daß man von Produkten ausgeht, die derart ausgewählt sind, daß man die Produkte der Formel (I) herstellt, worin $R_1$ einen Thiazol-2-yl-, 4-Methylthiazol-2-yl-, 4,5-Dimethylthiazol-2-yl-, 4-Ethylthiazol-2-yl-, 4-Trifluormethylthiazol-2-yl- oder 4-Ethoxycarbonylthiazol-2-yl-Rest bedeutet.

4. Verfahren gemäß einem der Ansprüche 1 bis 3, dadurch gekennzeichnet, daß man von einem Produkt der Formel (II) ausgeht, worin $R_2$ und $R_3$, die gleich oder voneinander verschieden sind, für einen Alkylrest mit 1 bis 3 Kohlenstoffatomen oder einen Allylrest stehen oder $R_2$ und $R_3$ gemeinsam den Rest $(CH_2)_4$ bilden und X ein Sauerstoffatom bedeutet.

5. Verfahren gemäß einem der Ansprüche 1 bis 4, dadurch gekennzeichnet, daß man 6-Ethyl-7-methoxy-5-methyl-2-(4-methylthiazol-2-yl)-imidazo[1,2-a]pyrimidin und 6-Ethyl-7-methoxy-5-methyl-2-(thiazol-2-yl)-imidazo[1,2-a]pyrimidin und deren Additionssalze mit Säuren herstellt.

6. Verfahren zur Herstellung pharmazeutischer Zusammensetzungen, dadurch gekennzeichnet, daß man als Wirkstoff zumindest eines der Produkte der Formel (I), erhalten gemäß Anspruch 1, oder zumindest eines ihrer pharmazeutisch verträglichen Salze in eine für die therapeutische Verwendung geeignete Form überführt.

7. Verfahren zur Herstellung pharmazeutischer Zusammensetzungen, dadurch gekennzeichnet, daß man als Wirkstoff zumindest eines der Produkte der Formel (I), erhalten gemäß einem der Ansprüche 2 bis 4, oder zumindest eines ihrer pharmazeutisch verträglichen Salze in eine für die therapeutische Verwendung geeignete Form überführt.

8. Verfahren zur Herstellung pharmazeutischer Zusammensetzungen, dadurch gekennzeichnet, daß man als Wirkstoff zumindest eines der Produkte der Formel (I), erhalten gemäß Anspruch 5, oder zumindest eines ihrer pharmazeutisch verträglichen Salze in eine für die therapeutische Verwendung geeignete Form überführt.

**Claims for contracting States: BE, CH, DE, FR, IT, LI, LU, NL, SE**

1. New 2-thiazolyl-imidazo-[1,2-a]-pyrimidines corresponding to formula (I):

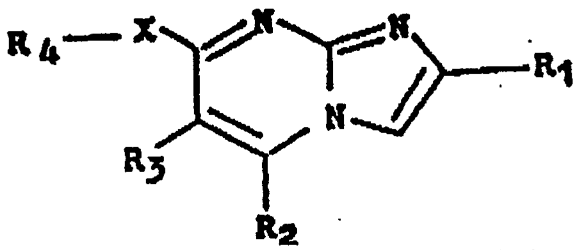

(I)

in which $R_1$ represents a 2-thiazolyl radical non-substituted or substituted either by one or two alkyl radicals containing form 1 to 3 carbon atoms, these radicals themselves being able to be non-substituted or substituted by one or more fluorine atoms, or by a $-CO_2Alk$ group in which Alk represents an alkyl radical containing from 1 to 3 carbon atoms, $R_2$ and $R_3$, identical or different, represent a hydrogen atom, an alkyl radical containing from 1 to 3 carbon atoms, an alkenyl radical containing from 1 to 3 carbon atoms, an alkenyl radical containing from 2 to 5 carbon atoms, or $R_2$ and $R_3$ together form a linear alkylene radical containing from 3 to 5 carbon atoms, X represents an oxygen or sulphur atom and $R_4$ represents an alkyl radical containing from 1 to 3 carbon atoms, as well as their addition salts with mineral or organic acids.

2. New 2-thiazolyl-imidazo-[1,2-a]-pyrimidines as defined in claim 1 and their addition salts with acids, characterised in that in the aforementioned formula (I), $R_1$ represents one of the following radicals, thiazol-2-yl, 4-methylthiazol-2-yl, 4,5-dimethylthiazol-2-yl, 4-ethylthiazol-2-yl, 4-trifluoromethylthiazol-2-yl or 4-ethoxycarbonylthiazol-2-yl.

3. New 2-thiazolyl-imidazo-[1,2-a]-pyrimidines as defined in claim 1 or 2 and their addition salts with acids, characterised in that in the aforementioned formula (I), $R_2$ and $R_3$, identical or different, represent an alkyl radical containing from 1 to 3 carbon atoms or an allyl radical or $R_2$ and $R_3$ together represent the $(CH_2)_4$ radical and X represents an oxygen atom.

4. Any one of the new 2-thiazolyl-imidazo-[1,2-a]-pyrimidines corresponding to formula I of claim 1, the names of which follow:

-6-ethyl-7-methoxy-5-methyl-2-(4-methylthiazol-2-yl)-imidazo-[1,2-a]-pyrimidine.
-6-ethyl-7-methoxy-5-methyl-2-(thiazol-2-yl)-imidazo-[1,2-a]-pyrimidine,
and their addition salts with acids.

5. Preparation process of new 2-thiazolyl-imidazo-[1,2-a]-pyrimidines as defined by fromula (I) of claim 1, as well as their addition salts with acids, characterised in that a product of formula (II):

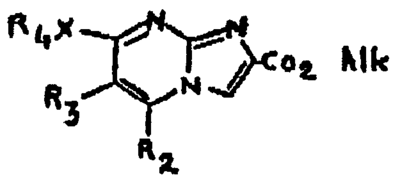

(II)

in which $R_2$, $R_3$, $R_4$ and X have the meaning already indicated, is treated by a basic agent then acidified so as to obtain a product of formula (III):

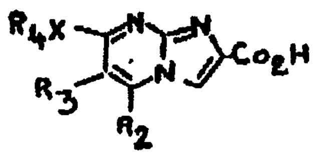

(III)

in which $R_2$, $R_3$, $R_4$ and X have the meaning already indicated, which product of formula (III) is treated by N,N'-carbonyldiimidazole to obtain a product of formula (IV):

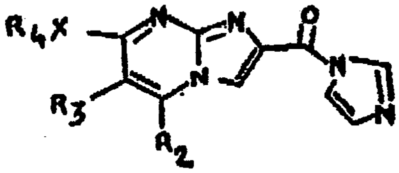

(IV)

in which $R_2$, $R_3$, $R_4$ and X have the meaning already indicated, which product of formula (IV) is treated with ammonia, to obtain a product of formula (V):

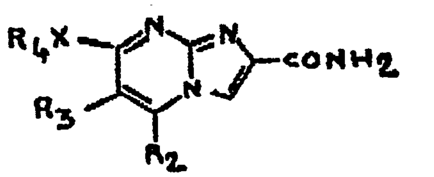

(V)

in which $R_2$, $R_3$, $R_4$ and X have the meaning already indicated, then the product of formula (V) is reacted with the Lawesson reagent or 2,4- bis-(4-methoxyphenyl)-1,3-dithia-2,4-diphosphetane-2,4-disulphide to obtain a product of formula (VI):

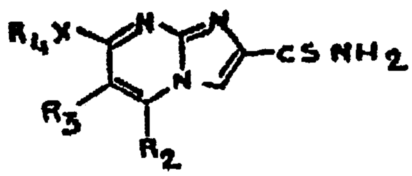

(VI)

in which $R_2$, $R_3$, $R_4$ and X have the meaning already indicated, then the product of formula (VI) is reacted with a product of formula (VII):

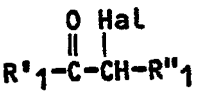

in which Hal represents a halogen atom, R'1 and R"1, identical or different, represent either a hydrogen atom, or an alkyl radical containing from 1 to 3 carbon atoms non-substituted or substituted by one or more fluorine atoms, or by a $-CO_2Alk$ radical in which Alk is defined as above, the intermediary obtained is treated by a basic agent to obtain a product of formula (I) which is isolated and if desired salified.

6. Preparation process according to claim 5, characterised in that:
- the basic agent that is reacted with the product of formula (II) is, for example, potassium carbonate and the reaction is effected in an aqueous mixture of an organic solvent such as methanol by heating to the reflux temperature of the reaction mixture;
- the reaction of the product of formula (III) with N,N'-carbonyldiimidazole is effected in an organic solvent, for example in dimethyl formamide;
- the treatment of the product of formula (IV) with ammonia is effected in an organic solvent such as chloroform;
- the reaction of the product of formula (V) with the Lawesson reagent is effected in an organic solvent such as tetrahydrofuran at the reflux temperature of the reaction medium;
- in formula (VII) Hal preferably represents a chlorine atom;
- the reaction of the product of formula (VI) with the product of formula (VII) is effected in an organic solvent such as ethanol and the intermediary obtained is subjected to a basic agent such as concentrated ammonium hydroxide.

7. Medicaments, characterised in that they are composed of new 2-thiazolyl-imidazo-[1,2-a]-pyrimidines as defined by formula (I) of claim 1, as well as by their addition salts with pharmaceutically acceptable acids.

8. Medicaments, characterised in that they are composed of new 2-thiazolyl-imidazo-[1,2-a]-pyrimidines as defined by any one of claims 2 or 3, as well as their addition salts with pharmaceutically acceptabl acids.

9. Medicaments, characterised in that they are composed of new 2-thiazolyl-imidazo-[1,2-a]-pyrimidines the names of which follow:
-6-ethyl-7-methoxy-5-methyl-2-(4-methylthiazol-2-yl)-imidazo-[1,2-a]-pyrimidine,
-6-ethyl-7-methoxy-5-methyl-2-(thiazol-2-yl)-imidazo-[1,2-a]-pyrimidine,
as well as their addition salts with pharmaceutically acceptable acids.

10. Pharmaceutical components, characterised in that they contain as active principle, at least one of the medicaments defined in any one of claims 7 to 9.

**Claims for contracting State: ES**

1. Preparation process for new 2-thiazolyl-imidazo-[1,2-a]-pyrimidines corresponding to formula (I):

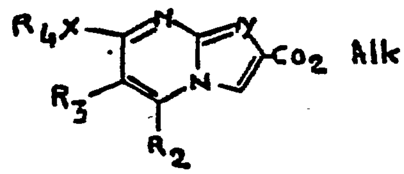

(II)

in which $R_1$ represents a 2-thiazoyl radical non-substituted or substituted by either one or two alkyl radicals containing from 1 to 3 carbon atoms, these radicals themselves being able to be non-substituted or substituted by one or more fluorine atoms, or by a $-CO_2Alk$ groupe in which Alk represents an alkyl radical containing from 1 to 3 carbon atoms, $R_2$ and $R_3$, identical or different, represent a hydrogen atom, an alkyl radical containing from 1 to 3 carbon atoms, an alkenyl radical containing from 2 to 5 carbon atoms, or $R_2$ and $R_3$ together form a linear alkylene radical containing from 3 to 5 carbon atoms, X represents an oxygen or sulphur atom and $R_4$ represents an alkyl radical containing from 1 to 3 carbon atoms, as well as their addition salts with mineral or organic acids, characterised in that a product of formula (II):

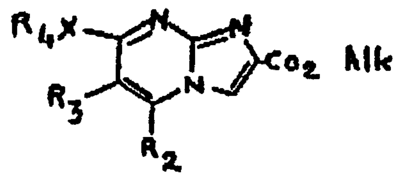

(II)

in which $R_2$, $R_3$, $R_4$ and X have the meaning already indicated, is treated with a basic agent then acidified so as to obtain a product of formula (III):

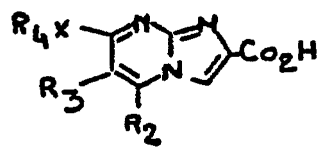

(III)

in which $R_2$, $R_3$, $R_4$ and X have the meaning already indicated, which product of formula (III) is treated with N,N'-carbonyldiimidazole to obtain a product of formula (IV):

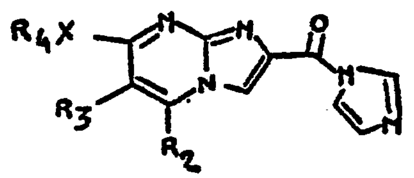

(IV)·

in which $R_2$, $R_3$, $R_4$ and X have the meaning already indicated, which product of formula (IV) is treated with ammonia, to obtain a product of formula (V):

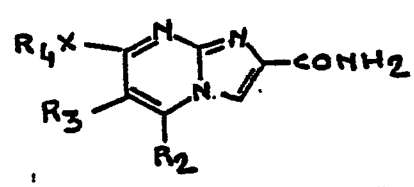

(V)

in which $R_2$, $R_3$, $R_4$ and X have the meaning already indicated, then the product of formula (V) is reacted with the Lawesson reagent or 2,4-bis-(4-methoxyphenyl)-1,3-dithia-2,4-diphosphetane-2,4-disulphide to obtain a product of formula (VI):

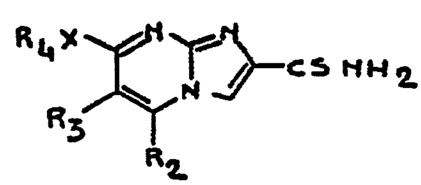

(VI)

in which $R_2$, $R_3$, $R_4$ and X have the meaning already indicated, then the product of formula (VI) is reacted with a product of formula (VII):

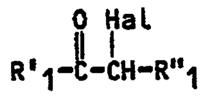

in which Hal represents a halogen atom, R'1 and R"1, identical or different, represent either a hydrogen atom, or an alkyl radical containing from 1 to 3 carbon atoms non-substituted or substituted by one or more fluorine atoms, or by a $-CO_2Alk$ radical in which Alk is defined as above, the intermediary obtained is treated by a basic reagent to obtain a product of formula (I) which is isolated and, if desired, salified.

2. Preparation process according to claim 1, characterised in that:

– the basic agent which is reacted with the product of formula (II) is, for example, potassium carbonate and the reaction is effected in an aqueous mixture of an organic solvent such as methanol, by heating to the reflux temperature of the reaction mixture;

– the reaction of the product of formula (III) with N,N'-carbonyldiimidazole is effected in an organic solvent, for example, in dimethyl formamide;

– the treatment of the product of formula (IV) with ammonia is effected in an organic solvent such as chloroform;

– the reaction of the product of formula (V) with the Lawesson reagent is effected in an organic solvent such as tetrahydrofuran at the reflux temperature of the reaction medium;

– in formula (VII) Hal preferably represents a chlorine atom;

– the reaction of the product of formula (VI) with the product of formula (VII) is effected in an organic solvent such as ethanol and the intermediary obtained is subjected to a basic agent such as concentrated ammonium hydroxide.

3. Preparation process according to claim 1 or 2, characterised in that products are chosen for initial employment in such a manner that the products of formula (I) are prepared and in which $R_1$ represents one of the following radicals, thiazol-2-yl, 4-methylthiazol-2-yl, 4,5-dimethylthiazol-2-yl, 4-ethylthiazol-2-yl, 4-trifluoromethylthiazol-2-yl or 4-ethoxycarbonyl-thiazol-2-yl.

4. Preparation process according to any one of claims 1 to 3, characterised in that to start with a product of formula (II) is used, in which $R_2$ and $R_3$, identical or different, represent an alkyl radical containing from 1 to 3 carbon atoms or an allyl radical or $R_2$ and $R_3$ together represent the $(CH_2)_4$ radical and X represents an oxygen atom.

**Claims for the contracting State: AT**

1. Preparation process for new 2-thiazolyl-imidazo-[1,2-a]-pyrimidines corresponding to formula (I):

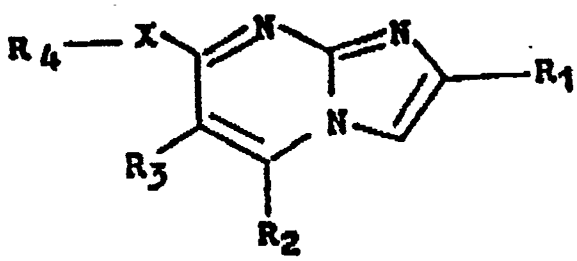

(I)

in which $R_1$ represents a 2-thiazoyl radical non-substituted or substituted by either one or two alkyl radicals containing from 1 to 3 carbon atoms, these radicals themselves being able to be non-substituted or substituted by one or more fluorine atoms, or by a $-CO_2Alk$ groupe in which Alk represents an alkyl radical containing from 1 to 3 carbon atoms, $R_2$ and $R_3$, identical or different, represent a hydrogen atom, an alkyl radical containing from 1 to 3 carbon atoms, an alkenyl radical containing from 2 to 5 carbon atoms, or $R_2$ and $R_3$ together form a linear alkylene radical containing from 3 to 5 carbon atoms, X represents an oxygen or sulphur atom and $R_4$ represents an alkyl radical containing from 1 to 3 carbon atoms, as well as their addition salts with mineral or organic acids, characterised in that a product of formula (II):

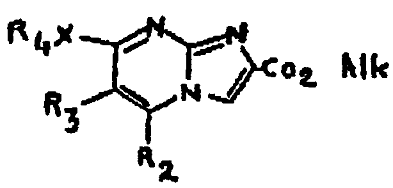

(II)

in which $R_2$, $R_3$, $R_4$ and X have the meaning already indicated, is treated with a basic agent then acidified

so as to obtain a product of formula (III):

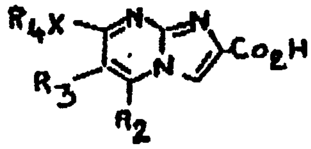 (III)

in which $R_2$, $R_3$, $R_4$ and X have the meaning already indicated, which product of formula (III) is treated with N,N'-carbonyldiimidazole to obtain a product of formula (IV):

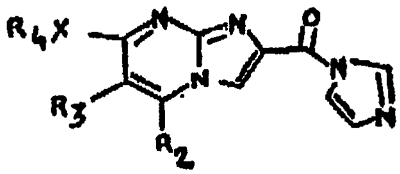 (IV)

in which $R_2$, $R_3$, $R_4$ and X have the meaning already indicated, which product of formula (IV) is treated with ammonia, to obtain a product of formula (V):

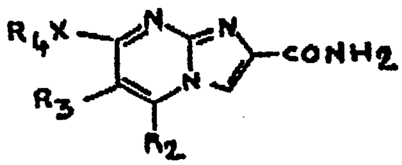 (V)

in which $R_2$, $R_3$, $R_4$ and X have the meaning already indicated, then the product of formula (V) is reacted with the Lawesson reagent or 2,4-bis-(4-methoxyphenyl)-1,3-dithia-2,4-diphosphetane-2,4-disulphide to obtain a product of formula (VI):

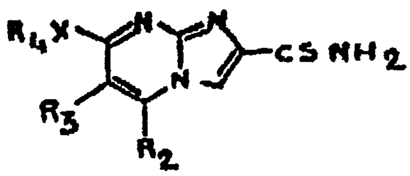 (VI)

in which $R_2$, $R_3$, $R_4$ and X have the meaning already indicated, then the product of formula (VI) is reacted with a product of formula (VII):

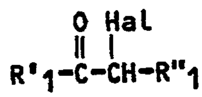

in which Hal represents a halogen atom, R'1 and R"1, identical or different, represent either a hydrogen atom, or an alkyl radical containing from 1 to 3 carbon atoms non-substituted or substituted by one or more fluorine atoms, or by a $-CO_2Alk$ radical in which Alk is defined as above, the intermediary obtained is treated by a basic reagent to obtain a product of formula (I) which is isolated and, if desired, salified.

2. Preparation process according to claim 1, characterised in that:
- the basic agent which is reacted with the product of formula (II) is, for example, potassium carbonate and the reaction is effected in an aqueous mixture of an organic solvent such as methanol, by heating to the reflux temperature of the reaction mixture;
- the reaction of the product of formula (III) with N,N'-carbonyldiimidazole is effected in an organic solvent, for example, in dimethyl formamide;
- the treatment of the product of formula (IV) with ammonia is effected in an organic solvent such as chloroform;
- the reaction of the product of formula (V) with the Lawesson reagent is effected in an organic solvent such as tetrahydrofuran at the reflux temperature of the reaction medium;
- in formula (VII) Hal preferably represents a chlorine atom;
- the reaction of the product of formula (VI) with the product of formula (VII) is effected in an organic solvent such as ethanol and the intermediary obtained is subjected to a basic agent such as concentrated ammonium hydroxide.

3. Preparation process according to claim 1 or 2, characterised in that products are chosen for initial employment in such a manner that the products of formula (I) are prepared and in which $R_1$ represents

one of the following radicals, thiazol-2-yl, 4-methylthiazol-2-yl, 4,5-dimethylthiazol-2-yl, 4-ethylthiazol-2-yl, 4-trifluoromethylthiazol-2-yl or 4-ethoxycarbonyl-thiazol-2-yl.

4. Preparation process according to any one of claims 1 to 3, characterised in that to start with a product of formula (II) is used, in which $R_2$ and $R_3$, identical or different, represent an alkyl radical containing from 1 to 3 carbon atoms or an allyl radical or $R_2$ and $R_3$ together represent the $(CH_2)_4$ radical and X represents an oxygen atom.

5. Preparation process according to any one of claims 1 to 4, characterised in that 6-ethyl-7-methoxy-5-methyl-2-(4-methylthiazol-2-yl)-imidazo-[1,2-a]-pyrimidine and 6-ethyl-7-methoxy-5-methyl-2-(thiazol-2-yl)-imidazo-[1,2-a]-pyrimidine and their addition salts with acids are prepared.

6. Preparation process of pharmaceutical compositions, characterised in that the active principle is at least one of the products of formula (I) as obtained in claim 1 or at least one of its pharmaceutically acceptable salts in a form adapted for therapeutic use.

7. Preparation process of pharmaceutical compositions, characterised in that the active principle is at least one of the products of formula (I) as obtained in any one of claims 2 to 4 or at least one of its pharmaceutically acceptable salts in a form adapted for therapeutic use.

8. Preparation process of pharmaceutical compositions, characterised in that the active principle is at least one of the products of formula (I) as obtained in claim 5 or at least one of its pharmaceutically acceptable salts in a form adapted for therapeutic use.

**Claims for the contracting State: GR**

1. Preparation process for new 2-thiazolyl-imidazo-[1,2-a]-pyrimidines corresponding to formula (I):

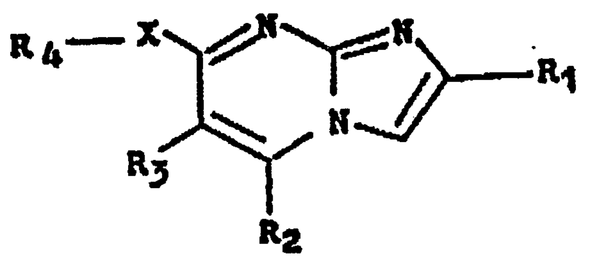

(I)

in which $R_1$ represents a 2-thiazoyl radical non-substituted or substituted by either one or two alkyl radicals containing from 1 to 3 carbon atoms, these radicals themselves being able to be non-substituted or substituted by one or more fluorine atoms, or by a $-CO_2Alk$ groupe in which Alk represents an alkyl radical containing from 1 to 3 carbon atoms, $R_2$ and $R_3$, identical or different, represent a hydrogen atom, an alkyl radical containing from 1 to 3 carbon atoms, an alkenyl radical containing from 2 to 5 carbon atoms, or $R_2$ and $R_3$ together form a linear alkylene radical containing from 3 to 5 carbon atoms, X represents an oxygen or sulphur atom and $R_4$ represents an alkyl radical containing from 1 to 3 carbon atoms, as well as their addition salts with mineral or organic acids, characterised in that a product of formula (II):

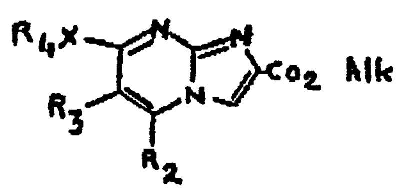

(II)

in which $R_2$, $R_3$, $R_4$ and X have the meaning already indicated, is treated with a basic agent then acidified so as to obtain a product of formula (III):

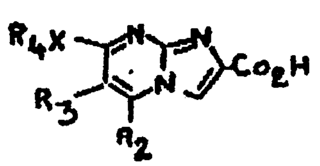

(III)

in which $R_2$, $R_3$, $R_4$ and X have the meaning already indicated, which product of formula (III) is treated with N,N'-carbonyldiimidazole to obtain a product of formula (IV):

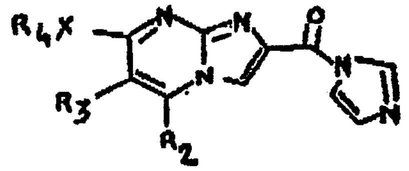

(IV)

in which $R_2$, $R_3$, $R_4$ and X have the meaning already indicated, which product of formula (IV) is treated with ammonia, to obtain a product of formula (V):

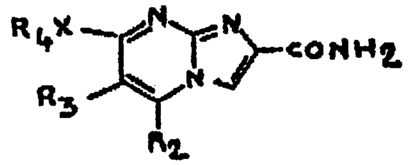

(V)

in which $R_2$, $R_3$, $R_4$ and X have the meaning already indicated, then the product of formula (V) is reacted with the Lawesson reagent or 2,4-bis-(4-methoxyphenyl)-1,3-dithia-2,4-diphosphetane-2,4-disulphide to obtain a product of formula (VI):

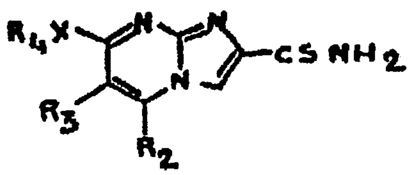

(VI)

in which $R_2$, $R_3$, $R_4$ and X have the meaning already indicated, then the product of formula (VI) is reacted with a product of formula (VII):

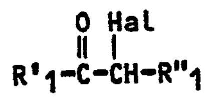

in which Hal represents a halogen atom, $R'_1$ and $R''_1$, identical or different, represent either a hydrogen atom, or an alkyl radical containing from 1 to 3 carbon atoms non-substituted or substituted by one or more fluorine atoms, or by a $-CO_2Alk$ radical in which Alk is defined as above, the intermediary obtained is treated by a basic reagent to obtain a product of formula (I) which is isolated and, if desired, salified.

2. Preparation process according to claim 1, characterised in that:
– the basic agent which is reacted with the product of formula (II) is, for example, potassium carbonate and the reaction is effected in an aqueous mixture of an organic solvent such as methanol, by heating to the reflux temperature of the reaction mixture;
– the reaction of the product of formula (III) with N,N'-carbonyldiimidazole is effected in an organic solvent, for example, in dimethyl formamide;
– the treatment of the product of formula (IV) with ammonia is effected in an organic solvent such as chloroform;
– the reaction of the product of formula (V) with the Lawesson reagent is effected in an organic solvent such as tetrahydrofuran at the reflux temperature of the reaction medium;
– in formula (VII) Hal preferably represents a chlorine atom;
– the reaction of the product of formula (VI) with the product of formula (VII) is effected in an organic solvent such as ethanol and the intermediary obtained is subjected to a basic agent such as concentrated ammonium hydroxide.

3. Preparation process according to claim 1 or 2, characterised in that products are chosen for initial employment in such a manner that the products of formula (I) are prepared and in which $R_1$ represents one of the following radicals, thiazol-2-yl, 4-methylthiazol-2-yl, 4,5-dimethylthiazol-2-yl, 4-ethylthiazol-2-yl, 4-trifluoromethylthiazol-2-yl or 4-ethoxycarbonyl-thiazol-2-yl.

4. Preparation process according to any one of claims 1 to 3, characterised in that to start with a product of formula (II) is used, in which $R_2$ and $R_3$, identical or different, represent an alkyl radical containing from 1 to 3 carbon atoms or an allyl radical or $R_2$ and $R_3$ together represent the $(CH_2)_4$ radical and X represents an oxygen atom.

5. Preparation process according to any one of claims 1 to 4, characterised in that 6-ethyl-7-methoxy-5-methyl-2-(4-methylthiazol-2-yl)-imidazo-[1,2-a]-pyrimidine and 6-ethyl-7-methoxy-5-methyl-2-(thiazol-2-yl)-imidazo-[1,2-a]-pyrimidine and their addition salts with acids are prepared.

6. Preparation process of pharmaceutical compositions, characterised in that the active principle is at least one of the products of formula (I) as obtained in claim 1 or at least one of its pharmaceutically acceptable salts in a form adapted for therapeutic use.

7. Preparation process of pharmaceutical compositions, characterised in that the active principle is at least one of the products of formula (I) as obtained in any one of claims 2 to 4 or at least one of its pharmaceutically acceptable salts in a form adapted for therapeutic use.

8. Preparation process of pharmaceutical compositions, characterised in that the active principle is at least one of the products of formula (I) as obtained in claim 5 or at least one of its pharmaceutically acceptable salts in a form adapted for therapeutic use.